(19) Europäisches Patentamt
European Patent Office
Office européen des brevets

(11) **EP 2 158 035 B1**

(12) **EUROPÄISCHE PATENTSCHRIFT**

(45) Veröffentlichungstag und Bekanntmachung des Hinweises auf die Patenterteilung:
**25.03.2020 Patentblatt 2020/13**

(21) Anmeldenummer: **08758905.7**

(22) Anmeldetag: **30.05.2008**

(51) Int Cl.:
*B01J 21/16* (2006.01)     *B01J 23/66* (2006.01)
*B01J 37/04* (2006.01)     *C07C 67/055* (2006.01)

(86) Internationale Anmeldenummer:
**PCT/EP2008/004335**

(87) Internationale Veröffentlichungsnummer:
**WO 2008/145394 (04.12.2008 Gazette 2008/49)**

(54) **ZIRKONIUMOXID-DOTIERTER KATALYSATORTRÄGER, VERFAHREN ZU DESSEN HERSTELLUNG SOWIE KATALYSATOR ENTHALTEND EINEN ZIRKONIUMOXID-DOTIERTEN KATALYSATORTRÄGER**

ZIRCONIUM-DOPED CATALYST SUPPORT, METHOD FOR PRODUCING THE SAME AND CATALYST CONTAINING A ZIRCONIUM-DOTED CATALYST SUPPORT

SUPPORT DE CATALYSEUR DOPÉ À L'OXYDE DE ZIRCONIUM, PROCÉDÉ DE PRODUCTION DUDIT SUPPORT DE CATALYSEUR ET CATALYSEUR CONTENANT UN SUPPORT DE CATALYSEUR DOPÉ À L'OXYDE DE ZIRCONIUM

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **31.05.2007 DE 102007025223**

(43) Veröffentlichungstag der Anmeldung:
**03.03.2010 Patentblatt 2010/09**

(73) Patentinhaber: **Süd-Chemie IP GmbH & Co. KG 81925 München (DE)**

(72) Erfinder:
• **HAGEMEYER, Alfred**
  **83043 Bad Aibling (DE)**
• **MESTL, Gerhard**
  **80935 München (DE)**
• **SCHECK, Peter**
  **82205 Gilching (DE)**

(74) Vertreter: **Kuba, Stefan et al**
  **Clariant Produkte (Deutschland) GmbH**
  **IPM / Patent & License Management**
  **Arabellastraße 4a**
  **81925 München (DE)**

(56) Entgegenhaltungen:
EP-A- 0 723 810     WO-A-00/58008
WO-A-99/22860     WO-A-2005/065821
DE-A1- 4 405 876     GB-A- 1 229 749
JP-A- H01 123 631     US-A- 2 656 323

**Beschreibung**

Die vorliegende Erfindung betriff einen porösen Katalysatorträger, bestehend aus einem ein natürliches Schichtsilikat umfassenden Material.

VAM ist ein wichtiger Monomerbaustein in der Synthese von Kunststoffpolymeren. Die Hauptanwendungsgebiete von VAM sind u.a. die Herstellung von Polyvinylacetat, Polyvinylalkohol und Polyvinylacetal sowie die Co- und Terpolymerisation mit anderen Monomeren wie zum Beispiel Ethylen, Vinylchlorid, Acrylat, Maleinat, Fumarat und Vinyllaurat.

VAM wird überwiegend in der Gasphase aus Essigsäure und Ethylen durch Umsetzung mit Sauerstoff hergestellt, wobei die für diese Synthese eingesetzten Katalysatoren vorzugsweise Pd und Au als Aktivmetalle enthalten sowie eine Alkalimetallkomponente als Promotor, vorzugsweise Kalium in Form des Acetats. In dem Pd/Au-System dieser Katalysatoren liegen die Aktivmetalle Pd und Au nicht in Form von Metallpartikeln des jeweiligen reinen Metalls vor, sondern vielmehr in Form von Pd/Au-Legierungspartikeln von möglicherweise unterschiedlicher Zusammensetzung, wenngleich das Vorliegen von unlegierten Partikeln nicht ausgeschlossen werden kann. Alternativ zu Au kann beispielsweise auch Cd oder Ba als zweite Aktivmetallkomponente eingesetzt sein.

Gegenwärtig wird VAM überwiegend mittels so genannter Schalenkatalysatoren hergestellt, bei welchen die katalytisch wirkenden Aktivmetalle des Katalysators den als Formkörper ausgebildeten Katalysatorträger nicht vollständig durchdringen, sondern vielmehr nur in einem mehr oder weniger breiten äußeren Bereich (Schale) des Katalysatorträger-Formkörpers enthalten sind (vgl. hierzu EP 565 952 A1, EP 634 214 A1, EP 634 209 A1 und EP 634 208 A1), während die weiter innen liegenden Bereiche des Trägers nahezu edelmetallfrei sind. Mit Hilfe von Schalenkatalysatoren ist in vielen Fällen eine selektivere Reaktionsführung möglich als mit Katalysatoren, bei denen die Träger bis in den Trägerkern mit den Aktivkomponenten imprägniert ("durchimprägniert") sind.

Die im Stand der Technik bekannten Schalenkatalysatoren zur Herstellung von VAM können beispielsweise Katalysatorträger auf der Basis von Siliziumoxid, Aluminiumoxid, Alumosilikat, Titanoxid oder Zirkoniumoxid sein (vgl. hierzu EP 839 793 A1, WO 1998/018553 A1, WO 2000/058008 A1 und WO 2005/061107 A1). Dabei gelangen Katalysatorträger auf der Basis von Titanoxid oder Zirkoniumoxid derzeit jedoch kaum zum Einsatz, da diese Katalysatorträger gegenüber Essigsäure nicht langzeitstabil bzw. verhältnismäßig teuer sind.

Die GB 1 229 749 beschreibt die Fällung von Zirkoniumverbindungen auf einem Kaolin-Ton, gefolgt von einer Sprühtrocknung des Materials. Das Material dient für Hydrierungsreaktionen in der Schwerölindustrie. Gegenstand der DE 44 05 876 A1 sind Katalysatorformkörper auf der Basis mindestens eines teilchenförmigen säureaktivierten Schichtsilikats, die dadurch gekennzeichnet sind, dass die Teilchen des säureaktivierten Schichtsilikats durch ein Bindemittel miteinander verbunden sind. Die in den Beispielen beschriebenen Katalysatorformkörper liegen in Form von Extrudaten vor.

In der EP 0 723 810 A1 wird ein Trägerkatalysator für die Produktion von Vinylacetatmonomer enthaltend auf einem Träger aus Siliziumdioxid, Alumosilikat oder Aluminiumoxid als katalytisch aktive Komponenten Palladium, Gold und Alkaliacetat, dadurch gekennzeichnet, dass der Träger zusätzlich mindestens ein Element aus den Gruppen IA, IIA, IIIA und IVB des Periodensystems der Elemente enthält.

Die in JP H01 123631 A beschriebene Herstellung eines zirkoniummodifizierten Montmorrilonits umfasst die Suspension des Montmorrilonits in einer zirkoniumhaltigen Lösung, gefolgt von einer Abtrennung des so erhaltenen Pulvers. Der so erhaltene Katalysator ist für Skelettisomerisierungen vorgesehen.

Katalysatoren gemäß US 2,656,323, die sich besonders für Olefinpolymerisierungen eignen sollen, werden durch die Vermengung einer Phosphorsäure, eines unkalzinierten siliziumhaltigen Adsorbents und einer Verbindung eines Elements der Gruppe IVB des Periodensystems der Elemente bereitgestellt.

Der überwiegende Anteil der gegenwärtig eingesetzten Katalysatoren zur Herstellung von VAM sind Schalenkatalysatoren mit einer Pd/Au-Schale auf einem porösen amorphen, als Kugel ausgebildeten Alumosilikatträger auf der Basis von natürlichen Schichtsilikaten in Form von natürlichen säurebehandelten kalzinierten Bentoniten, die mit Kaliumacetat als Promotor durchimprägniert sind.

Derartige VAM-Schalenkatalysatoren werden üblicherweise auf so genanntem chemischen Wege hergestellt, bei welchem der Katalysatorträger mit Lösungen von entsprechenden Metall-Vorläuferverbindungen beispielsweise durch Eintauchen des Trägers in die Lösungen oder mittels des Incipient-Wetness-Verfahrens (Porenfüllverfahren), bei welchem der Träger mit einem seinem Porenvolumen entsprechenden Lösungsvolumen beladen bzw. getränkt wird. Die Pd/Au-Schale des Katalysators wird beispielsweise erzeugt, indem zunächst der Katalysatorträger-Formkörper in einem ersten Schritt mit einer $Na_2PdCl_4$-Lösung getränkt und anschließend in einem zweiten Schritt die Pd-Komponente mit NaOH-Lösung auf den Katalysatorträger in Form einer Pd-Hydroxidverbindung fixiert wird. In einem darauffolgenden, separaten dritten Schritt wird der Katalysatorträger dann mit einer $NaAuCl_4$-Lösung getränkt und danach die Au-Komponente ebenfalls mittels NaOH fixiert. Nach der Fixierung der Edelmetallkomponente in einer äußeren Schale des Katalysatorträgers wird der beladene Katalysatorträger dann weitestgehend frei von Chlorid- und Na-Ionen gewaschen, anschließend getrocknet, kalziniert und abschließend bei 150 °C mit Ethylen reduziert. Die so erzeugte Pd/Au-Schale weist üblicherweise eine Dicke von etwa 100 bis 500 μm auf.

Üblicherweise wird der mit den Edelmetallen beladene Katalysatorträger nach dem Fixierungs- oder Reduzierungsschritt mit Kaliumacetat beladen, wobei die Beladung mit Kaliumacetat nicht nur in der äußeren, mit Edelmetallen beladenen Schale erfolgt, sondern der Katalysatorträger vielmehr mit dem Promotor vollständig durchimprägniert wird. Als Katalysatorträger wird überwiegend ein kugelförmiger Träger mit der Bezeichnung "KA-160" der SÜD-Chemie AG auf der Basis von natürlichen säurebehandelten kalzinierten Bentoniten als Schichtsilikat eingesetzt, der eine BET-Oberfläche von ungefähr 160 m$^2$/g aufweist.

Die mittels der im Stand der Technik bekannten VAM-Schalenkatalysatoren auf der Basis von Pd und Au als Aktivmetalle und KA-160-Trägern als Katalysatorträger erreichten Selektivitäten von VAM betragen circa 90 Mol.-% bezogen auf das zugeführte Ethylen, wobei die verbleibenden 10 Mol.-% der Reaktionsprodukte im Wesentlichen $CO_2$ sind, das durch Totaloxidation der organischen Edukte/Produkte gebildet wird.

Zur Steigerung der Aktivität dieser Katalysatoren wurden die aktivmetallfreien Katalysatorträger-Formkörper auf der Basis von natürlichen Schichtsilikaten vor der Edelmetallabscheidung zunächst mit Zirkoniumoxid oberflächendotiert. Dazu wurde beispielsweise ein fertig ausgebildeter Formkörper auf Bentonit-Basis mit einer Lösung einer Zirkoniumoxid-Vorläuferverbindung imprägniert und anschließend die Vorläuferverbindung durch Kalzination des Formkörpers in das entsprechende Oxid überführt. Derartige Katalysatoren zeichnen sich zwar im Vergleich zu den entsprechenden im Stand der Technik bekannten Katalysatoren mit einer Pd/Au-Schale durch eine gesteigerte Aktivität hinsichtlich der VAM-Produktion aus, die Aktivität kann jedoch nur in einem beschränkten Umfang erhöht werden, da das $ZrO_2$ Poren belegt, in welchen auch Pd und Au der Oxidationsstufe 0 abzuscheiden sind. Bei einer Überladung des Trägers mit $ZrO_2$ kann eine Abnahme der Aktivität des Katalysators beobachtet werden.

Aufgabe der vorliegenden Erfindung ist es daher, einen Katalysatorträger bereitzustellen, mittels dem VAM-Katalysatoren hergestellt werden können, die sich durch eine verhältnismäßig hohe VAM-Aktivität auszeichnen. Diese Aufgabe wird gelöst durch einen porösen Katalysatorträger, bestehend aus einem ein natürliches Schichtsilikat umfassenden Material, wobei $ZrO_2$ in dem Material verteilt enthalten ist, wobei es sich bei dem natürlichen Schichtsilikat um einen säurebehandelten, kalzinierten Bentonit handelt, wobei das $ZrO_2$ gleichmäßig verteilt enthalten ist und das $ZrO_2$ in partikulärer Form vorliegt, der Katalysatorträger als Kugel ausgebildet ist und eine Härte von größer/gleich 20 N aufweist Überraschenderweise wurde aufgefunden, dass durch Inkorporation von $ZrO_2$ in ein ein natürliches Schichtsilikat umfassendes Material, aus welchem die Matrix des porösen Trägers gebildet ist, Katalysatorträger erhältlich sind, mittels denen VAM-Katalysatoren hergestellt werden können, die sich durch eine verhältnismäßig hohe VAM-Aktivität auszeichnen.

Darüber hinaus zeichnen sich mittels des Katalysatorträgers hergestellte VAM-Katalysatoren durch eine verhältnismäßig hohe VAM-Selektivität aus und neigen über verhältnismäßig lange Standzeiten hinweg nur geringfügig zur thermischen Alterung.

Ferner zeichnet sich der erfindungsgemäße Katalysatorträger durch eine hohe chemische Resistenz in der VAM-Synthese aus. Während das in die Schichtsilikat-Matrix eingebaute $ZrO_2$ weitgehend beständig gegen Essigsäure ist und daher der Träger eine höhere Langzeitstabilität sowie damit einhergehend ein entsprechender Katalysator eine hohe Aktivität über lange Standzeiten hinweg aufweist, wird das oberflächenimprägnierte $ZrO_2$ bei Trägern des Standes der Technik verhältnismäßig schnell in Zirkonylacetat, welches keine Promotorwirkung in der VAM-Synthese zeigt, umgewandelt und ausgewaschen.

Darüber hinaus ist der erfindungsgemäße Katalysatorträger besonders kostengünstig. Die Herstellung des erfindungsgemäßen Katalysatorträgers beinhaltet lediglich einen Kalzinierschritt, in welchem beispielsweise ein Gemisch eines pulverförmigen Schichtsilikats und einer pulverförmigen Zirkoniumoxid-Vorläuferverbindung kalziniert wird, und ist somit diesbezüglich kostengünstiger als eine Oberflächendotierung eines bereits kalzinierten Trägers mit Zirkoniumoxid-Vorläuferverbindungen mit nachfolgender zweiter Kalzinierung oder das Aufbringen von Zirkondioxid oder Zirkoniumoxid-Vorläuferverbindungen als Suspensionen auf bereits kalzinierte Träger (WO2005065821) und deren nachträgliches Kalzinieren, wobei dieses Verfahren sogar noch einen zusätzlichen Binder, üblicherweise Zirkonylacetat, benötigt, um eine ausreichende Festigkeit der Katalysatorträger zur erhalten.

Ein mittels des erfindungsgemäßen Katalysatorträgers hergestellter VAM-Katalysator ist durch eine besonders hohe VAM-Aktivität und -Selektivität gekennzeichnet, wenn das $ZrO_2$ in dem Material gleichmäßig verteilt enthalten ist, vorzugsweise homogen.

Das $ZrO_2$ liegt in dem Material in partikulärer Form vor. Dadurch wird eine feste Inkorporation des $ZrO_2$ in dem Material und damit eine hohe thermische Alterungsbeständigkeit eines mittels des erfindungsgemäßen Katalysatorträgers hergestellten Katalysators gewährleistet. Die $ZrO_2$-Partikel weisen dabei vorzugsweise einen mittleren Durchmesser von 1 nm bis 100 μm auf, bevorzugt einen mittleren Durchmesser 0,5 μm bis 20 μm.

Im Rahmen der vorliegenden Erfindung werden die Begriffe "Katalysatorträger-Formkörper", "Katalysatorträger", "Formkörper" und "Träger" synonym verwendet.

Bevorzugt ist es, dass die Zirkoniumoxidpartikel in dem Katalysatorträger mit einem Anteil von 1 bis 25 Mass.-% enthalten sind, vorzugsweise mit einem Anteil von 3 bis 20 Mass.-% und bevorzugt mit einem Anteil von 5 bis 20 Mass.-% bezogen auf die Masse des Katalysatorträgers. Ist das Zirkoniumoxid mit einem Anteil von weniger als 1 Mass.-% in dem Katalysatorträger vertreten, so wirken sich die die Aktivität steigernden Eigenschaften des Zirkoniumoxids nur geringfügig aus, während oberhalb eines Anteils von 25 Mass.-% die Steigerung der Aktivität des Katalysators mit einer deutlichen Abnahme der VAM-Selektivität einhergehen kann.

Entsprechend einer bevorzugten Ausführungsform des erfindungsgemäßen Katalysatorträgers ist seine Löslichkeit in Essigsäure kleiner als 8 Gew.-%, bevorzugt kleiner als 4 Gew.-% und besonders bevorzugt kleiner als 2 Gew.-%. Zur Bestimmung der Essigsäurelöslichkeit werden 5 g des Katalysatorträgers zu Pulver gemahlen und in 125 ml 96 %ige Essigsäure (p.a.) für 1 h unter Rückflussbedingungen behandelt. Der Katalysatorträger wird über einen Filter abgetrennt. Das Eluat wird zur Trockene eingedampft und die Masse m(Rückstand) des Feststoffrückstands bestimmt und die Essigsäurelöslichkeit wie folgt berechnet: Essigsäurelöslichkeit = (m(Rückstand)/5g)*100 %.

Kleine Löslichkeitswerte des erfindungsgemäßen Katalysatorträgers können erhalten werden, indem der Katalysatorträger nach seiner Herstellung mit Säure behandelt wird. Entsprechend ist gemäß einer weiter bevorzugten Ausführungsform der Katalysatorträger ein mit einer Säure behandelter Träger.

Ferner ist es bevorzugt, wenn der Katalysatorträger eine Azidität von zwischen 1 und 150 $\mu$val/g aufweist, vorzugsweise eine von zwischen 5 und 130 $\mu$val/g, bevorzugt eine von zwischen 10 und 100 $\mu$val/g und besonders bevorzugt eine von zwischen 10 und 60 $\mu$val/g.

Die Azidität des Katalysatorträgers kann die Aktivität des erfindungsgemäßen Katalysators bei der Gasphasensynthese von VAM aus Essigsäure und Ethen vorteilhaft beeinflussen. Die Azidität des Katalysatorträgers wird dabei wie folgt bestimmt: 1 g des fein gemahlenen Katalysatorträgers wird mit 100 ml Wasser (mit einem pH-Blindwert) versetzt und unter Rühren 15 Minuten extrahiert. Anschließend wird mit 0,01 n NaOH-Lösung zumindest bis pH 7,0 titriert, wobei die Titration stufenweise erfolgt; und zwar wird zunächst 1 ml der NaOH-Lösung zu dem Extrakt getropft (1 Tropfen/Sekunde), dann 2 Minuten gewartet, der pH-Wert abgelesen, erneut 1 ml NaOH zugetropft, usw. Der Blindwert des eingesetzten Wassers wird bestimmt und die Azidäts-Berechnung entsprechend korrigiert.

Die Titrationskurve (ml 0,01 NaOH gegen pH-Wert) wird dann aufgetragen und der Schnittpunkt der Titrationskurve bei pH 7 bestimmt. Berechnet werden die Moläquivalente in $10^{-6}$ äquiv/g Träger, die sich aus dem NaOH-Verbrauch für den Schnittpunkt bei pH 7 ergeben.

$$\text{Gesamtsäure:} \qquad \frac{10 * ml\ 0,01\ n\ NaOH}{1\ Träger} = \mu val/g$$

Darüber hinaus ist es bevorzugt, dass der Katalysatorträger einen mittleren Porendurchmesser von 8 bis 30 nm aufweist, vorzugsweise einen von 9 bis 20 nm und bevorzugt einen von 10 bis 15 nm.

Es wurde festgestellt, dass die VAM-Selektivität des erfindungsgemäßen Katalysators umso höher ist, je kleiner die Oberfläche des erfindungsgemäßen Katalysatorträgers ist. Unter dem Begriff "Oberfläche" des Katalysatorträgers wird dabei im Rahmen der vorliegenden Erfindung die BET-Oberfläche des Trägers verstanden, die mittels Adsorption von Stickstoff nach DIN 66132 bestimmt wird.

Daher kann es bevorzugt sein, dass der Katalysatorträger eine BET-Oberfläche von kleiner/gleich 145 $m^2$/g aufweist, vorzugsweise eine von kleiner/gleich 142 $m^2$/g, bevorzugt eine von kleiner/gleich 140 $m^2$/g, weiter bevorzugt eine von kleiner/gleich 137 $m^2$/g, mehr bevorzugt eine von kleiner/gleich 135 $m^2$/g, noch mehr bevorzugt eine von kleiner/gleich 133 $m^2$/g und besonders bevorzugt eine von kleiner/gleich 130 $m^2$/g.

Erfindungsgemäß ist es ferner bevorzugt, dass der Katalysatorträger eine BET-Oberfläche von 60 bis 145 $m^2$/g aufweist, vorzugsweise eine von zwischen 65 und 140 $m^2$/g, bevorzugt eine von zwischen 70 und 135 $m^2$/g, weiter bevorzugt eine von zwischen 70 und 120 $m^2$/g, mehr bevorzugt eine von zwischen 70 und 110 $m^2$/g und am meisten bevorzugt eine von zwischen 70 und 100 $m^2$/g.

Der Katalysatorträger weist eine Härte von größer/gleich 20 N auf, vorzugsweise eine von größer/gleich 25 N, weiter bevorzugt eine von größer/gleich 35 N und am meisten bevorzugt eine von größer/gleich 40 N. Die Härte (Druckhärte) ist dabei wie nachstehend ausgeführt zu bestimmen.

Unter dem Begriff "natürliches Schichtsilikat", wofür in der Literatur auch der Begriff "Phyllosilikat" verwendet wird, wird im Rahmen der vorliegenden Erfindung aus natürlichen Quellen stammendes, behandeltes Silikat-Mineral verstanden, in welchem $SiO_4$-Tetraeder, welche die strukturelle Grundeinheit aller Silikate bilden, in Schichten der allgemeinen Formel $[Si_2O_5]^{2-}$ miteinander vernetzt sind. Diese Tetraederschichten wechsellagern mit so genannten Oktaederschichten, in denen ein Kation, vor allem Al und Mg, oktaedrisch von OH bzw. 0 umgeben ist. Dabei werden beispielsweise Zweischicht-Phyllosilikate und Dreischicht-Phyllosilikate unterschieden. Definitionen des Begriffes "Schichtsilikate" finden sich beispielsweise in "Lehrbuch der anorganischen Chemie", Hollemann Wiberg, de Gruyter, 102. Auflage, 2007 (ISBN 978-3-11-017770-1) oder in "Römpp Lexikon Chemie", 10. Auflage, Georg Thieme Verlag unter dem Begriff "Phyllosilikat". Typische Behandlungen, denen ein natürliches Schichtsilikat vor dem Einsatz als Trägermaterial unterzogen wird, beinhalten beispielsweise ein Behandeln mit Säuren und/oder ein Kalzinieren. Das natürliche Schichtsilikat im Rahmen der vorliegenden Erfindung ist ein säurebehandelter, kalzinierter Bentonit. Bentonite sind zwar im eigentlichen Sinne keine natürlichen Schichtsilikate, sondern vielmehr ein Gemisch von überwiegend Tonmineralien, in welchem Schichtsilikate enthalten sind. Vorliegend ist zu verstehen, dass es sich bei dem natürlichen Schichtsilikat in dem Katalysatorträger um einen säurebehandelten und kalzinierten Bentoniten handelt.

Bevorzugt ist es, dass der Anteil des Katalysatorträgers an natürlichem Schichtsilikat größer/gleich 50 Mass.-% ist, vorzugsweise größer/gleich 60 Mass.-%, bevorzugt größer/gleich 70 Mass.-%, weiter bevorzugt größer/gleich 80 Mass.-%, mehr bevorzugt größer/gleich 90 Mass.-% und am meisten bevorzugt größer/gleich 93 Mass.-% bezogen auf die Masse des Katalysatorträgers.

Es konnte festgestellt werden, dass die VAM-Selektivität eines VAM-Katalysators von dem integralen Porenvolumen des erfindungsgemäßen Katalysatorträgers abhängig ist. Daher ist es bevorzugt, dass der Katalysatorträger ein integrales Porenvolumen nach BJH von zwischen 0,25 und 0,7 ml/g aufweist, vorzugsweise eines von zwischen 0,3 und 0,55 ml/g und bevorzugt eines von 0,35 bis 0,5 ml/g.

Dabei ist das integrale Porenvolumen des Katalysatorträgers nach der Methode von BJH mittels Stickstoffadsorption bestimmt. Die Oberfläche des Katalysatorträgers sowie sein integrales Porenvolumen werden nach der BET- bzw. nach der BJH-Methode bestimmt. Die Bestimmung der BET-Oberfläche erfolgt nach der BET-Methode gemäß DIN 66131; eine Veröffentlichung der BET-Methode findet sich auch in J. Am. Chem. Soc. 60, 309 (1938). Zur Bestimmung der Oberfläche und des integralen Porenvolumens des Katalysatorträgers oder des Katalysators kann die Probe beispielsweise mit einem vollautomatischen Stickstoffporosimeter der Firma Micromeritics, Typ ASAP 2010 vermessen werden, mittels dessen eine Adsorptions- sowie Desorptionsisotherme aufgenommen wird. Zur Ermittlung der Oberfläche und der Porosität des Katalysatorträgers nach der BET-Theorie werden die Daten gemäß DIN 66131 ausgewertet. Das Porenvolumen wird aus den Messdaten unter Anwendung der BJH-Methode ermittelt (E.P. Barret, L.G. Joiner, P.P. Haienda, J. Am. Chem. Soc. 73 (1951, 373)). Bei diesem Verfahren werden auch Effekte der Kapillarkondensation berücksichtigt. Porenvolumina bestimmter Porengrößenbereiche werden durch Aufsummieren inkrementeller Porenvolumina bestimmt, die aus der Auswertung der Adsorptionsisotherme nach BJH erhalten werden. Das integrale Porenvolumen nach der BJH-Methode bezieht sich auf Poren mit einem Durchmesser von 1,7 bis 300 nm.

Entsprechend einer weiter bevorzugten Ausführungsform des erfindungsgemäßen Katalysatorträgers ist es bevorzugt, wenn zumindest 80 % des integralen Porenvolumens des Katalysatorträgers von Mesoporen und Makroporen gebildet sind, vorzugsweise zumindest 85 % und bevorzugt zumindest 90 %. Dadurch wird einer durch Diffusionslimitierung bewirkten, verminderten Aktivität des erfindungsgemäßen Katalysators entgegengewirkt, insbesondere bei Pd/Au-Schalen mit verhältnismäßig großen Dicken. Dabei sollen diesbezüglich unter den Begriffen "Mikroporen", "Mesoporen" und "Makroporen" Poren verstanden werden, die einen Durchmesser von kleiner als 2 nm, einen Durchmesser von 2 bis 50 nm bzw. einen Durchmesser von größer als 50 nm aufweisen.

Auch bevorzugt ist es, wenn der Katalysatorträger eine Schüttdichte von mehr als 0,3 g/ml aufweist, vorzugsweise eine von mehr als 0,35 g/ml und besonders bevorzugt eine Schüttdichte von zwischen 0,35 und 0,6 g/ml.

Ferner bevorzugt ist es, wenn das im Träger enthaltene, natürliche Schichtsilikat einen $SiO_2$-Gehalt von zumindest 65 Mass.-% aufweist, vorzugsweise einen von zumindest 80 Mass.-% und bevorzugt einen von 95 bis 99,5 Mass.-%. Dadurch wird eine ausreichende chemische Beständigkeit des erfindungsgemäßen Katalysatorträgers gewährleistet.

Bei der Gasphasensynthese von VAM aus Essigsäure und Ethen wirkt sich ein verhältnismäßig niedriger $Al_2O_3$-Gehalt in dem natürlichen Schichtsilikat kaum nachteilig aus, während bei hohen $Al_2O_3$-Gehalten mit einer merklichen Abnahme der Druckhärte gerechnet werden muss. Gemäß einer bevorzugten Ausführungsform des erfindungsgemäßen Katalysatorträgers enthält das natürliche Schichtsilikat daher weniger als 10 Mass.-% $Al_2O_3$, vorzugsweise 0,1 bis 3 Mass.-% und bevorzugt 0,3 bis 1,0 Mass.-% bezogen auf die Masse des natürlichen Schichtsilikats.

Der Katalysatorträger ist als Kugel ausgebildet.

Darüber hinaus ist es bevorzugt, dass der Katalysatorträger als Kugel mit einem Durchmesser von größer als 2 mm ausgebildet ist, vorzugsweise mit einem Durchmesser von größer als 3 mm und bevorzugt mit einem Durchmesser von größer als 4 mm.

Darüber hinaus ist es bevorzugt, dass die maximale Abmessung des Katalysatorträgers kleiner als 25 mm ist, vorzugsweise kleiner als 10 mm.

Auch kann es zur Erhöhung der Aktivität eines VAM-Katalysators bevorzugt sein, dass der erfindungsgemäße Katalysatorträger mit zumindest einem Oxid eines Metalls dotiert ist, ausgewählt aus der Gruppe bestehend aus Hf, Ti, Nb, Ta, W, Mg, Re, Y und Fe, vorzugsweise mit $HfO_2$ oder $Fe_2O_3$. In diesem Zusammenhang ist es ferner bevorzugt, dass der Anteil des Katalysatorträgers an Dotierungsoxid zwischen 0 und 20 Mass.-% beträgt, vorzugsweise 1,0 bis 10 Mass.-% und bevorzugt 3 bis 8 Mass.-%. Die Dotierung kann dabei beispielsweise durch Oberflächen-Dotierung, wie sie aus dem Stand der Technik bekannt ist, erfolgen oder das Metalloxid/die Metalloxide kann/können in die Matrix des Katalysatorträgers eingearbeitet sein wie das $ZrO_2$ des erfindungsgemäßen Katalysatorträgers.

In dem erfindungsgemäßen Katalysatorträger liegen die Zirkoniumoxidpartikel bevorzugt in Form von Mikrokristalliten und/oder Nanokristalliten vor, wobei das Zirkoniumoxid darin nicht zwingend als reines $ZrO_2$ vorliegt, sondern

auch in Form eines Mischoxides vorliegen kann.

Es kann bevorzugt sein, dass die $ZrO_2$-Partikel selbst mit $Y_2O_3$ oder $HfO_2$ dotiert sind.

Gemäß einer weiter bevorzugten Ausführungsform kann es vorgesehen sein, dass die Wassersaugfähigkeit des Katalysatorträgers 40 bis 75 % beträgt, bevorzugt 50 bis 70 % berechnet als Gewichtszunahme durch Wasseraufnahme. Die Saugfähigkeit wird bestimmt, indem 10 g der Trägerprobe mit entionisiertem Wasser 30 min lang getränkt wird, bis von der Trägerprobe keine Gasblasen mehr entweichen. Dann wird das überschüssige Wasser dekantiert und die getränkte Probe mit einem Baumwolltuch abgetupft zur Befreiung der Probe von anhaftender Feuchtigkeit. Anschließend wird der wasserbeladene Träger ausgewogen und die Saugfähigkeit berechnet gemäß:

```
(Auswaage (g) - Einwaage (g)) x 10 = Wassersaugfähigkeit (%)
```

Die vorliegende Erfindung betrifft ferner ein Verfahren zur Herstellung eines erfindungsgemäßen Katalysatorträgers.

Der erfindungsgemäße Katalysatorträger kann beispielsweise hergestellt werden, indem ein pulverförmiger (unkalzinierter) säurebehandelter Bentonit als Schichtsilikat mit einer pulverförmigen Zirkoniumverbindung sowie Wasser vermahlen und dann innig bis zur Homogenität vermischt wird, die resultierende Mischung unter Verdichtung zu einem Formkörper mittels dem Fachmann geläufiger Vorrichtungen, wie beispielsweise Extrudern oder Tablettenpressen, geformt wird und anschließend der nicht ausgehärtete Formkörper zu einem stabilen Formkörper kalziniert wird. Die Kalzinierung wird dabei bei Temperaturen durchgeführt, bei welchen sich ein festes Gefüge ergibt sowie ggf. die Zirkoniumverbindung in Zirkoniumoxid $ZrO_2$ überführt wird. Dabei hängt die Größe der spezifischen Oberfläche (BET) des Katalysatorträgers insbesondere von der Qualität des eingesetzten (Roh-)Bentonits ab, dem Säurebehandlungsverfahren des eingesetzten Bentonits, d.h. beispielsweise der Natur und der zum Bentonit relativen Menge und der Konzentration der eingesetzten anorganischen Säure, der Säurebehandlungsdauer sowie der -temperatur, vom Verpressungsdruck sowie von der Kalzinierdauer und -temperatur sowie der Kalzinieratmosphäre.

Säurebehandelte Bentonite können durch Behandlung von Bentoniten mit starken Säuren erhalten werden, wie beispielsweise Schwefelsäure, Phosphorsäure oder Salzsäure. Eine auch im Rahmen der vorliegenden Erfindung geltende Definition des Begriffes Bentonit ist in Römpp, Lexikon Chemie, 10. Aufl., Georg Thieme Verlag, angegeben. Im Rahmen der vorliegenden Erfindung besonders bevorzugte Bentonite sind natürliche aluminiumhaltige Schichtsilikate, die Montmorillonit (als Smektit) als Hauptmineral enthalten. Nach der Säurebehandlung wird der Bentonit in der Regel mit Wasser gewaschen, getrocknet und zu einem Pulver vermahlen.

Das erfindungsgemäße Verfahren zur Herstellung eines erfindungsgemäßen Katalysatorträgers, umfasst die Schritte:

a) Vermischen eines pulverförmigen säurebehandelten Bentonits, mit pulverförmigem Zirkonmetall oder einer pulverförmigen Zirkoniumverbindung;

b) Kalzinieren des erhaltenen Gemisches beziehungsweise des Formkörpers bei einer Temperatur von 400 bis 800°C;

c) Formen eines Formkörpers aus dem erhaltenen Gemisch, vorzugsweise vor Schritt b).

Die Herstellung eines Katalysatorträgers gemäß dem erfindungsgemäßen Verfahren beinhaltet somit lediglich einen einzigen Kalzinierschritt und ist somit diesbezüglich kostengünstiger als eine Oberflächendotierung eines bereits kalzinierten Trägers mit Zirkoniumoxid-Vorläuferverbindungen mit nachfolgender zweiter Kalzinierung oder das Aufbringen von Zirkondioxid oder Zirkoniumoxid-Vorläuferverbindungen als Suspensionen auf bereits kalzinierte Träger (WO2005065821) und deren nachträgliches Kalzinieren, wobei dieses Verfahren sogar noch einen zusätzlichen Binder, üblicherweise Zirkonylacetat, benötigt, um genügend Festigkeit der Katalysatorträger zur erhalten.

Das Kalzinieren wird bei einer Temperatur von 400 °C bis 800 °C durchgeführt, bevorzugt bei einer Temperatur von 500 °C bis 700 °C.

Während der Kalzinierung die Zirkoniumverbindung, sofern diese nicht schon Zirkoniumoxid ist, in ein Oxid überführt.

Aus dem vorgenannten Verfahren resultieren Katalysatorträger mit einem festen Gefüge von miteinander zusammengesintertem Schichtsilikat und Zirkoniumoxidpartikeln, wobei die Zirkoniumoxidpartikel in dem Gefüge der Partikel gleichmäßig verteilt sind.

In das vorgenannte Verfahren werden als pulverförmige Zirkoniumverbindung vorzugsweise Zirkoniumdioxid, Zirkoniumhydroxid, Zirkonylacetat oder andere Zirkonylcarboxylate, Zirkoniumcarbonat oder Zirkoniumoxicarbonat, Zirkonylnitrat, Zirkoniumnaphthenat oder Ammoniumzirkoncarbonat eingesetzt, vorzugsweise Zirkoniumhydroxid. Beim

Einsatz von $ZrO_2$ kann dieses mit $Y_2O_3$ und/oder $HfO_2$ stabilisiert sein. Dadurch wird ein Sintern der Bestandteile der Matrix des Katalysatorträger-Formkörpers zu einem festen Gefüge von Schichtsilikat und ggf. Zirkoniumoxidpartikeln gewährleistet.

Entsprechend einer weiter bevorzugten Ausführungsform des erfindungsgemäßen Verfahrens umfasst das Verfahren ferner den Schritt: Behandeln des kalzinierten Gemisches mit einer Säure. Dadurch wird eine geringe Essigsäurelöslichkeit des Trägers beim Einsatz in der VAM-Synthese erreicht Alternativ zu einer pulverförmigen Zirkoniumverbindung kann auch in einer Lösung befindliches kolloidales $ZrO_2$ oder ein entsprechendes Sol eingesetzt werden.

In dem erfindungsgemäßen Verfahren ist es vorgesehen, dass die Zirkoniumverbindung bei der Kalzinierung in ein Oxid überführt wird, sofern diese nicht vor der Kalzinierung schon als Zirkoniumoxid vorlag.

Entsprechend einer bevorzugten Ausführungsform des erfindungsgemäßen Verfahrens ist es vorgesehen, dass als Zirkoniumverbindung Zirkoniumoxid und/oder Zirkoniumhydroxid eingesetzt wird.

Auch kann es vorgesehen sein, dass in Schritt a) desweiteren $Y_2O_3$ und/oder $HfO_2$ eingesetzt wird.

Die vorliegende Erfindung betrifft ferner einen Schalenkatalysator für die Herstellung von VAM, umfassend den erfindungsgemäßenKatalysatorträger, in dessen äußerer Schale metallisches Pd und Au enthalten ist.

Entsprechend einer bevorzugten Ausführungsform des erfindungsgemäßen Katalysators ist es vorgesehen, dass die Zirkoniumoxidpartikel in dem Katalysatorträger mit einem Anteil von 1 bis 25 Mass.-% enthalten sind, vorzugsweise mit einem Anteil von 3 bis 20 Mass.-% und bevorzugt mit einem Anteil von 5 bis 20 Mass.-% bezogen auf die Masse des Katalysatorträgers.

Gemäß einer weiter bevorzugten Ausführungsform des erfindungsgemäßen Katalysators ist es vorgesehen, dass der Katalysatorträger eine Azidität von zwischen 1 und 150 $\mu$val/g aufweist, vorzugsweise eine von zwischen 5 und 130 $\mu$val/g, bevorzugt eine von zwischen 10 und 100 $\mu$val/g und besonders bevorzugt eine von zwischen 10 und 60 $\mu$val/g.

Entsprechend einer weiter bevorzugten Ausführungsform des erfindungsgemäßen Katalysators ist es vorgesehen, dass der Katalysatorträger einen mittleren Porendurchmesser von 8 bis 30 nm aufweist, vorzugsweise einen von 9 bis 20 nm und bevorzugt einen von 10 bis 15 nm.

Gemäß einer weiter bevorzugten Ausführungsform des erfindungsgemäßen Katalysators ist es vorgesehen, dass der Katalysatorträger eine Oberfläche von kleiner/gleich 145 m$^2$/g aufweist, vorzugsweise eine von kleiner als 142 m$^2$/g, bevorzugt eine von kleiner als 140 m$^2$/g, weiter bevorzugt eine von kleiner als 137 m$^2$/g, mehr bevorzugt eine von kleiner als 135 m$^2$/g, noch mehr bevorzugt eine von kleiner als 133 m$^2$/g und besonders bevorzugt eine von kleiner als 130 m$^2$/g.

Entsprechend einer bevorzugten Ausführungsform des erfindungsgemäßen Katalysators ist es vorgesehen, dass der Katalysatorträger eine Oberfläche von 60 m$^2$/g bis 145 m$^2$/g aufweist, vorzugsweise eine von zwischen 65 m$^2$/g und 140 m$^2$/g, bevorzugt eine von zwischen 70 m$^2$/g und 130 m$^2$/g, weiter bevorzugt eine von zwischen 70 m$^2$/g und 120 m$^2$/g, mehr bevorzugt eine von zwischen 70 m$^2$/g und 110 m$^2$/g und am meisten bevorzugt eine von zwischen 70 m$^2$/g und 100 m$^2$/g.

Insbesondere um den Abrieb des erfindungsgemäßen Katalysators in vertretbaren Grenzen zu halten, weist der Katalysator eine Härte von größer/gleich 20 N auf, vorzugsweise eine von größer/gleich 25 N, weiter bevorzugt eine von größer/gleich 35 N und am meisten bevorzugt eine von größer/gleich 40 N. Die Ermittlung der Härte ist dabei mittels eines Tablettenhärtetesters 8M der Fa. Dr. Schleuniger Pharmatron AG an 99 Stück Schalenkatalysatoren als Durchschnitt bestimmt nach Trocknung des Katalysators bei 130 °C für 2h, wobei die Geräteeinstellungen wie folgt sind:

| | |
|---|---|
| Härte: | N |
| Distanz zum Formkörper: | 5,00 mm |
| Zeitverzögerung: | 0,80 s |
| Vorschub-Typ: | 6 D |
| Geschwindigkeit: | 0,60 mm/s |

Die Härte des Katalysators kann beispielsweise mittels Variation gewisser Parameter des Verfahrens zur Herstellung des Katalysatorträgers beeinflusst werden, beispielsweise durch die Auswahl des natürlichen Schichtsilikats, die Kalzinierdauer und/oder die Kalziniertemperatur eines aus der entsprechenden Trägermischung geformten, unausgehärteten Formkörpers, oder durch bestimmte Zuschlagsstoffe wie beispielsweise Methylcellulose oder Magnesiumstearat.

Entsprechend einer weiter bevorzugten Ausführungsform des erfindungsgemäßen Katalysators ist es vorgesehen, dass der Anteil des Katalysatorträgers an säurebehandeltem kalziniertem Bentonit, größer/gleich 50 Mass.-% ist, vorzugsweise größer/gleich 60 Mass.-%, bevorzugt größer/gleich 70 Mass.-%, weiter bevorzugt größer/gleich 80 Mass.-%, mehr bevorzugt größer/gleich 90 Mass.-% und am meisten bevorzugt größer/gleich 93 Mass.-% bezogen auf die Masse des Katalysatorträgers.

Gemäß einer weiter bevorzugten Ausführungsform des erfindungsgemäßen Katalysators ist es vorgesehen, dass der Katalysatorträger ein integrales Porenvolumen nach BJH von zwischen 0,25 und 0,7 ml/g aufweist, vorzugsweise eines von zwischen 0,3 und 0,55 ml/g und bevorzugt eines von 0,35 bis 0,5 ml/g.

Entsprechend einer weiter bevorzugten Ausführungsform des erfindungsgemäßen Katalysators ist es vorgesehen, dass zumindest 80 % des integralen Porenvolumens des Katalysatorträgers von Mesoporen und Makroporen gebildet sind, vorzugsweise zumindest 85 % und bevorzugt zumindest 90 %.

Gemäß einer weiter bevorzugten Ausführungsform des erfindungsgemäßen Katalysators ist es vorgesehen, dass der Katalysatorträger eine Schüttdichte von mehr als 0,3 g/ml aufweist, vorzugsweise eine von mehr als 0,35 g/ml und besonders bevorzugt eine Schüttdichte von zwischen 0,35 und 0,6 g/ml.

Entsprechend einer weiter bevorzugten Ausführungsform des erfindungsgemäßen Katalysators ist es vorgesehen, dass das im Träger enthaltene natürliche Schichtsilikat einen $SiO_2$-Gehalt von zumindest 65 Mass.-% aufweist, vorzugsweise einen von zumindest 80 Mass.-% und bevorzugt einen von 95 bis 99,5 Mass.-%.

Gemäß einer weiter bevorzugten Ausführungsform des erfindungsgemäßen Katalysators ist es vorgesehen, dass das im Träger enthaltene natürliche Schichtsilikat weniger als 10 Mass.-% $Al_2O_3$ enthält, vorzugsweise 0,1 bis 3 Mass.-% und bevorzugt 0,3 bis 1,0 Mass.-%.

Der Katalysatorträger ist als Kugel ausgebildet.

Gemäß einer weiter bevorzugten Ausführungsform des erfindungsgemäßen Katalysators ist es vorgesehen, dass der Katalysatorträger als Kugel mit einem Durchmesser von größer als 2 mm ausgebildet ist, vorzugsweise mit einem Durchmesser von größer als 3 mm und bevorzugt mit einem Durchmesser von größer als 4 mm.

Darüber hinaus ist es bevorzugt, dass die maximale Abmessung des Katalysatorträgers kleiner als 25 mm ist, vorzugsweise kleiner als 10 mm.

Gemäß einer weiter bevorzugten Ausführungsform des erfindungsgemäßen Katalysators ist es vorgesehen, dass der Katalysatorträger mit zumindest einem Oxid eines Metalls dotiert ist, ausgewählt aus der Gruppe bestehend aus Hf, Ti, Nb, Ta, W, Mg, Re, Y und Fe, vorzugsweise mit $HfO_2$ oder $Fe_2O_3$.

Entsprechend einer weiter bevorzugten Ausführungsform des erfindungsgemäßen Katalysators ist es vorgesehen, dass der Anteil des Katalysatorträgers an Dotierungsoxid zwischen 0 und 20 Mass.-% beträgt, vorzugsweise 1,0 bis 10 Mass.-% und bevorzugt 3 bis 8 Mass.-%.

Die VAM-Selektivität des erfindungsgemäßen Katalysators ist im Allgemeinen umso höher, je kleiner die Dicke der Pd/Au-Schale des Katalysators ist. Entsprechend einer weiter bevorzugten Ausführungsform des erfindungsgemäßen Katalysators weist daher die Schale des Katalysators eine Dicke von kleiner/gleich 300 $\mu$m auf, vorzugsweise eine von kleiner/gleich 200 $\mu$m, bevorzugt eine von kleiner/gleich 150 $\mu$m, weiter bevorzugt eine von kleiner/gleich 100 $\mu$m und mehr bevorzugt eine von kleiner/gleich 80 $\mu$m.

Die Dicke der Schale kann mittels eines Mikroskops optisch ausgemessen werden. Und zwar erscheint der Bereich, in dem die Edelmetalle abgeschieden sind, schwarz, während die edelmetallfreien Bereiche weiß erscheinen. Die Grenzlinie zwischen edelmetallhaltigen und -freien Bereichen ist in der Regel sehr scharf und optisch deutlich zu erkennen. Sollte die vorgenannte Grenzlinie nicht scharf ausgebildet und entsprechend optisch nicht deutlich zu erkennen sein, so entspricht die Dicke der Schale der Dicke einer Schale, gemessen ausgehend von der äußeren Oberfläche des Katalysatorträgers, in welcher 95 % des auf dem Träger abgeschiedenen Edelmetalls enthalten sind.

Es konnte ebenfalls festgestellt werden, dass bei dem erfindungsgemäßen Katalysator die Pd/Au-Schale mit einer eine hohe Aktivität des Katalysators bewirkenden, verhältnismäßig großen Dicke ausgebildet werden kann, ohne eine nennenswerte Verminderung der VAM-Selektivität des erfindungsgemäßen Katalysators zu bewirken. Entsprechend einer anderen bevorzugten Ausführungsform des erfindungsgemäßen Katalysators weist die Schale des Katalysators daher eine Dicke von zwischen 200 und 2000 $\mu$m auf, vorzugsweise eine von zwischen 250 und 1800 $\mu$m, bevorzugt eine von zwischen 300 und 1500 $\mu$m und weiter bevorzugt eine von zwischen 400 und 1200 $\mu$m.

Um eine ausreichende Aktivität des erfindungsgemäßen Katalysators zu gewährleisten, beträgt der Anteil des Katalysators an Pd 0,5 bis 2,5 Mass.-%, vorzugsweise 0,6 bis 2,3 Mass.-% und bevorzugt 0,7 bis 2 Mass.-% bezogen auf die Masse des mit Edelmetall beladenen Katalysatorträgers.

Ferner kann es bevorzugt sein, wenn der erfindungsgemäße Katalysator einen Pd-Gehalt von 1 bis 20 g/l aufweist, vorzugsweise einen von 2 bis 15 g/l und bevorzugt einen von 3 bis 10 g/l.

Ebenfalls um eine ausreichende Aktivität und Selektivität des erfindungsgemäßen Katalysators zu gewährleisten, liegt das Au/Pd-Atomverhältnis des Katalysators vorzugsweise zwischen 0 und 1,2, vorzugsweise zwischen 0,1 und 1, bevorzugt zwischen 0,3 und 0,9 und besonders bevorzugt zwischen 0,4 und 0,8.

Darüber hinaus kann es bevorzugt sein, wenn der Au-Gehalt des erfindungsgemäßen Katalysators von 1 bis 20 g/l beträgt, vorzugsweise 1,5 bis 15 g/l und bevorzugt 2 bis 10 g/l.

Um eine weitgehend einheitliche Aktivität des erfindungsgemäßen Katalysators über die Dicke der Pd/Au-Schale hinweg zu gewährleisten, sollte die Edelmetallkonzentration über die Schalendicke hinweg nur verhältnismäßig wenig variieren. Bevorzugt ist es daher, wenn das Profil der Edelmetallkonzentration des Katalysators über einen Bereich von 90 % der Schalendicke hinweg, wobei der Bereich zur äußeren und inneren Schalengrenze jeweils um 5 % der Schalendicke beabstandet ist, von der mittleren Edelmetallkonzentration dieses Bereichs um maximal +/- 20 % abweicht, vorzugsweise um maximal +/- 15 % und bevorzugt um maximal +/- 10 %. Derartige Profile sind mittels des weiter unten beschriebenen Aufsprühens auf ein Fließbett von Trägern erhältlich.

Chlorid vergiftet den erfindungsgemäßen Katalysator und führt zu einer Deaktivierung desselben. Entsprechend einer weiter bevorzugten Ausführungsform des erfindungsgemäßen Katalysators ist daher sein Gehalt an Chlorid kleiner/gleich 250 ppm, vorzugsweise kleiner/gleich 150 ppm.

Der erfindungsgemäße Katalysator enthält neben Zirkoniumoxid als weiteren Promotor vorzugsweise zumindest eine Alkalimetallverbindung, vorzugsweise eine Kalium-, eine Natrium-, eine Cäsium- oder eine Rubidiumverbindung, bevorzugt eine Kaliumverbindung. Zu den geeigneten und besonders bevorzugten Kaliumverbindungen gehören Kaliumacetat KOAc, Kaliumcarbonat $K_2CO_3$, Kaliumhydrogencarbonat $KHCO_3$ und Kaliumhydroxid KOH sowie sämtliche Kaliumverbindungen, die sich unter den jeweiligen Reaktionsbedingungen der VAM-Synthese in K-Acetat KOAc umwandeln. Die Kaliumverbindung kann sowohl vor als auch nach der Reduktion der Metall-Komponenten zu den Metallen Pd und Au auf den Katalysatorträger aufgetragen werden. Entsprechend einer weiter bevorzugten Ausführungsform des erfindungsgemäßen Katalysators umfasst der Katalysator ein Alkalimetallacetat, vorzugsweise Kaliumacetat. Dabei ist es zur Gewährleistung einer ausreichenden Promotoraktivität besonders bevorzugt, wenn der Gehalt des Katalysators an Alkalimetallacetat 0,1 bis 0,7 mol/l beträgt, vorzugsweise 0,3 bis 0,5 mol/l. Im Falle von Kaliumacetat beträgt der Gehalt des erfindungsgemäßen Katalysators an Kaliumacetat vorzugsweise etwa 40 g/l.

Entsprechend einer weiter bevorzugten Ausführungsform des erfindungsgemäßen Katalysators beträgt das Alkalimetall/Pd-Atomverhältnis zwischen 1 und 12, vorzugsweise zwischen 2 und 10 und besonders bevorzugt zwischen 4 und 9. Dabei ist vorzugsweise das Alkalimetall/Pd-Atomverhältnis umso geringer, je kleiner die Oberfläche des Katalysatorträgers ist.

Die vorliegende Erfindung betrifft ferner ein Verfahren zur Herstellung insbesondere eines Schalenkatalysators, umfassend die Schritte:

a) Bereitstellen des erfindungsgemäßen Katalysatorträgers;

b) Auftragen einer Lösung einer Pd-Vorläuferverbindung auf den Katalysatorträger;

c) Auftragen einer Lösung einer Au-Vorläuferverbindung auf den Katalysatorträger;

d) Überführen der Pd-Komponente der Pd-Vorläuferverbindung in die metallische Form; und

e) Überführen der Au-Komponente der Au-Vorläuferverbindung in die metallische Form.

Die mit $ZrO_2$ dotierte äußere Schale des erfindungsgemäßen Katalysatorträgers weist dabei eine Dicke auf, die zumindest der Dicke der aufzutragenden Edelmetallschale entspricht, beispielsweise eine Dicke von 500 $\mu$m.

Grundsätzlich kann als Pd- und Au-Vorläuferverbindung jede Pd- bzw. Au-Verbindung eingesetzt werden, mittels derer ein hoher Dispersionsgrad der Metalle erzielt werden kann. Dabei wird unter dem Begriff "Dispersionsgrad" das Verhältnis der Anzahl aller Oberflächenmetallatome aller Metall-/Legierungspartikel eines geträgerten Metallkatalysators zu der Gesamtzahl aller Metallatome der Metall-/Legierungspartikel verstanden. Im Allgemeinen ist es bevorzugt, wenn der Dispersionsgrad einem verhältnismäßig hohen Zahlenwert entspricht, da in diesem Fall möglichst viele Metallatome für eine katalytische Reaktion frei zugänglich sind. Das heißt, dass bei einem verhältnismäßig hohen Dispersionsgrad eines geträgerten Metallkatalysators eine bestimmte katalytische Aktivität desselben mit einer verhältnismäßig geringen Menge an eingesetztem Metall erreicht werden kann. Entsprechend einer weiter bevorzugten Ausführungsform des erfindungsgemäßen Katalysators beträgt der Dispersionsgrad der Metallpartikel 1 bis 30 %. Dabei sind die Werte des Dispersionsgrads mittels CO-Adsorbtion gemäß der entsprechenden DIN-Norm bestimmt.

Es kann bevorzugt sein, dass die Pd- und Au-Vorläuferverbindungen ausgewählt sind aus den Halogeniden, insbesondere Chloriden, Oxiden, Nitraten, Nitriten, Formiaten, Propionaten, Oxalaten, Acetaten, Hydroxiden, Hydrogencarbonaten, Aminkomplexen oder organischen Komplexen, beispielsweise Triphenylphosphinkomplexen oder Acetylacetonatkomplexen, dieser Metalle.

Beispiele für bevorzugte Pd-Vorläuferverbindungen sind wasserlösliche Pd-Salze. Gemäß einer besonders bevorzugten Ausführungsform des erfindungsgemäßen Verfahrens ist die Pd-Vorläuferverbindung ausgewählt aus der Gruppe bestehend aus $Pd(NH_3)_4(OH)_2$, $Pd(NH_3)_4(OAc)_2$, $H_2PdCl_4$, $Pd(NH_3)_4(HCO_3)_2$, $Pd(NH_3)_4(HPO_4)$, $Pd(NH_3)_4Cl_2$, $Pd(NH_3)_4$-Oxalat, Pd-Oxalat, $Pd(NO_3)_2$, $Pd(NH_3)_4(NO_3)_2$, $K_2Pd(OAc)_2(OH)_2$, $Na_2Pd(OAc)_2(OH)_2$, $Pd(NH_3)_2(NO_2)_2$, $K_2Pd(NO_2)_4$, $Na_2Pd(NO_2)_4$, $Pd(OAc)_2$, $K_2PdCl_4$, $(NH_4)_2PdCl_4$, $PdCl_2$ und $Na_2PdCl_4$. Anstelle von $NH_3$ können auch die entsprechenden Komplexsalze mit Ethylendiamin oder Ethanolamin als Ligand verwendet werden. Neben $Pd(OAc)_2$ können auch andere Carboxylate des Palladiums eingesetzt werden, vorzugsweise die Salze der aliphatischen Monocarbonsäuren mit 3 bis 5 Kohlenstoffatomen, beispielsweise das Propionat- oder das Butyratsalz.

Entsprechend einer weiter bevorzugten Ausführungsform des erfindungsgemäßen Verfahrens können auch Pd-Nitrit-Vorläuferverbindungen bevorzugt sein. Bevorzugte Pd-Nitrit-Vorläuferverbindungen sind beispielsweise solche, die mittels Lösen von $Pd(OAc)_2$ in einer $NaNO_2$-Lösung erhalten werden. Beispiele für bevorzugte Au-Vorläuferverbindungen sind wasserlösliche Au-Salze. Gemäß einer besonders bevorzugten Ausführungsform des erfindungsgemäßen Verfahrens ist die Au-Vorläuferverbindung ausgewählt aus der Gruppe bestehend aus $KAuO_2$, $HAuCl_4$, $KAu(NO_2)_4$, $AuCl_3$, $KAuCl_4$, $NaAuCl_4$, $KAuCl_4$, $KAu(OAc)_3(OH)$, $HAu(NO_3)_4$, $NaAuO_2$, $NMe_4AuO_2$, $RbAuO_2$, $CsAuO_2$, $NaAu(OAc)_3(OH)$, $RbAu(OAc)_3OH$, $CsAu(OAc)_3OH$, $NMe_4Au(OAc)_3OH$ und $Au(OAc)_3$. Dabei ist es gegebenenfalls empfehlenswert $Au(OAc)_3$ oder $KAuO_2$ mittels Fällung des Oxids/Hydroxids aus einer Goldsäure-Lösung, Waschung und Isolierung des Niederschlags sowie Aufnahme desselben in Essigsäure bzw. KOH jeweils frisch anzusetzen.

Als Lösungsmittel für die Vorläuferverbindungen sind alle reinen Lösungsmittel oder Lösungsmittelgemische geeignet, in denen die ausgewählten Vorläuferverbindungen löslich sind und die nach dem Auftrag auf den Katalysatorträger von demselben leicht mittels Trocknung wieder entfernt werden können. Bevorzugte Lösungsmittel für Metallacetate als Vorläuferverbindungen sind beispielsweise Aceton oder unsubstituierte Carbonsäuren, insbesondere Essigsäure, und für die Metallchloride vor allem Wasser oder verdünnte Salzsäure.

Falls die Vorläuferverbindungen in reinen Lösungsmitteln wie Aceton, Essigsäure, Wasser bzw. verdünnter Salzsäure oder Mischungen davon nicht ausreichend löslich sind, können alternativ oder zusätzlich zu den genannten Lösungsmitteln auch andere Lösungsmittel oder Lösungsmitteladditive Anwendung finden. Als andere Lösungsmittel kommen hierbei vorzugsweise diejenigen Lösungsmittel in Betracht, die inert sind und mit Essigsäure oder Wasser mischbar sind. Als bevorzugte Lösungsmittel, die sich als Zusatz zur Essigsäure eignen, seien Ketone, beispielsweise Aceton oder Acetylaceton, ferner Ether, beispielsweise Tetrahydrofuran oder Dioxan, Acetonitril, Dimethylformamid und Lösungsmittel auf der Basis von Kohlenwasserstoffen wie beispielsweise Benzol genannt.

Als bevorzugte Lösungsmittel oder Additive, die sich als Zusatz zu Wasser eignen, seien Ketone, beispielsweise Aceton, oder Alkohole, beispielsweise Ethanol oder Isopropanol oder Methoxyethanol, Laugen, wie wässrige KOH oder NaOH, oder organische Säuren, wie Essigsäure, Ameisensäure, Zitronensäure, Weinsäure, Äpfelsäure, Glyoxylsäure, Glycolsäure, Oxalsäure, Oxamsäure, Glycin, Benztraubensäure oder Milchsäure, genannt.

Werden als Vorläuferverbindungen Chloridverbindungen eingesetzt, so muss sichergestellt werden, dass die Chloridionen vor dem Einsatz des nach dem erfindungsgemäßen Verfahren hergestellten Katalysators auf eine tolerable Restmenge reduziert werden, da Chlorid ein Katalysatorgift ist. Dazu wird der Katalysatorträger im Regelfall nach der Fixierung der Pd- und Au-Komponente der Pd- bzw. Au-Vorläuferverbindung auf den Katalysatorträger ausgiebig mit Wasser gewaschen. Dies geschieht im Allgemeinen entweder unmittelbar nach der Fixierung durch Hydroxid-Fällung der Pd- und Au-Komponente mittels Lauge oder nach der Reduktion der Edelmetall-Komponente zu dem/der jeweiligen Metall/Legierung.

Gemäß einer bevorzugten Ausführungsform des erfindungsgemäßen Verfahrens werden jedoch chloridfreie Pd- und Au-Vorläuferverbindungen verwendet sowie chloridfreie Lösungsmittel, um den Gehalt des Katalysators an Chlorid möglichst gering zu halten und ein aufwendiges "Chloridfrei-Waschen" zu vermeiden. Dabei werden vorzugsweise als Vorläuferverbindungen die entsprechenden Acetat-, Hydroxid- oder Nitritverbindungen eingesetzt, da diese den Katalysatorträger nur in einem sehr geringen Umfang mit Chlorid kontaminieren.

Die Abscheidung der Pd- und Au-Vorläuferverbindungen auf den Katalysatorträger im Bereich einer äußeren Schale des Katalysatorträgers lässt sich nach an sich bekannten Verfahren erzielen. So kann der Auftrag der Vorläufer-Lösungen durch Tränkung erfolgen, indem der Träger in die Vorläufer-Lösungen eingetaucht wird oder gemäß dem Incipient-Wetness-Verfahren getränkt wird. Anschließend wird auf den Katalysatorträger eine Base, beispielsweise Natronlauge oder Kalilauge, aufgetragen, wodurch die Edelmetall-Komponenten in Form von Hydroxiden auf den Träger ausgefällt werden. Es ist beispielsweise auch möglich, den Träger zunächst mit Lauge zu tränken und dann die Vorläuferverbindungen auf den so vorbehandelten Träger aufzubringen.

Gemäß einer weiter bevorzugten Ausführungsform des erfindungsgemäßen Verfahrens ist es daher vorgesehen, dass die Pd- und die Au-Vorläuferverbindung auf den Katalysatorträger aufgetragen wird, indem der Katalysatorträger mit der Lösung der Pd-Vorläuferverbindung und mit der Lösung der Au-Vorläuferverbindung oder mit einer Lösung, die sowohl die Pdals auch die Au-Vorläuferverbindung enthält, getränkt wird.

Nach dem Stand der Technik werden die Aktivmetalle Pd und Au ausgehend von Chloridverbindungen im Bereich einer Schale des Trägers auf demselben mittels Tränken aufgebracht. Diese Technik ist jedoch an die Grenzen angelangt, was minimale Schalendicken und maximale Au-Beladung angeht. Die Schalendicke der entsprechenden bekannten VAM-Katalysatoren liegt bestenfalls bei ca. 100 $\mu m$ und es ist nicht absehbar, dass mittels Tränkung noch dünnere Schalen erhalten werden können. Darüber hinaus lassen sich höhere Au-Beladungen innerhalb der gewünschten Schale mittels Tränkung nur schwerlich realisieren, da die Au-Vorläuferverbindungen dazu neigen, von der Schale in innere Zonen des Katalysatorträger-Formkörpers zu diffundieren, was zu breiten Au-Schalen führt, die bereichsweise kaum mit Pd durchmischt sind.

Die Aktivmetalle, oder, anders gesagt, deren Vorläuferverbindungen, können beispielsweise auch mittels so genannter physikalischer Verfahren auf den Träger aufgetragen werden. Dazu kann der Träger erfindungsgemäß bevorzugt beispielsweise mit einer Lösung der Vorläuferverbindungen besprüht werden, wobei der Katalysatorträger in einer Dragiertrommel bewegt wird, in welcher warme Luft eingeblasen wird, so dass das Lösungsmittel rasch verdampft.

Entsprechend einer weiter bevorzugten Ausführungsform des erfindungsgemäßen Verfahrens ist es aber vorgesehen, dass die Lösung der Pd-Vorläuferverbindung und die Lösung der Au-Vorläuferverbindung auf den Katalysatorträger aufgetragen wird, indem die Lösungen auf eine Wirbelschicht oder ein Fließbett des Katalysatorträgers aufgesprüht werden, vorzugsweise mittels eines Aerosols der Lösungen. Dadurch kann die Schalendicke stufenlos eingestellt und optimiert werden, beispielsweise bis zu einer Dicke von 2 mm. Aber auch sehr dünne Edelmetallschalen mit einer Dicke von kleiner als 100 $\mu$m sind so möglich.

Bevorzugt ist es, wenn die Formkörper in dem Fließbett elliptisch oder toroidal umlaufen. Um eine Vorstellung davon zu geben, wie sich die Formkörper in derartigen Fließbetten bewegen, sei ausgeführt, dass bei "elliptischem Umlaufen" sich die Katalysatorträger-Formkörper in dem Fließbett in vertikaler Ebene auf einer elliptischen Bahn bewegen mit wechselnder Größe der Haupt- und Nebenachse. Bei "toroidalem Umlaufen" bewegen sich die Katalysatorträger-Formkörper in dem Fließbett in vertikaler Ebene auf einer elliptischen Bahn mit wechselnder Größer der Haupt- und Nebenachse und in horizontaler Ebene auf einer Kreisbahn mit wechselnder Größe des Radius. Im Mittel bewegen sich die Formkörper bei "elliptischem Umlaufen" in vertikaler Ebene auf einer elliptischen Bahn, bei "toroidalem Umlaufen" auf einer toroidalen Bahn, d.h., dass ein Formkörper die Oberfläche eines Torus mit vertikal elliptischem Schnitt helikal abfährt.

Die vorstehend genannte Ausführungsform des erfindungsgemäßen Verfahrens wird vorzugsweise mittels eines Fließbettes in einer Fließbettanlage durchgeführt. Besonders bevorzugt ist es dabei, wenn in der Anlage eine so genannte kontrollierte Luftgleitschicht besteht. Zum Einen werden die Katalysatorträger-Formkörper durch die kontrollierte Luftgleitschicht gut durchmischt, wobei sie gleichzeitig um ihre eigene Achse rotieren, wodurch sie gleichmäßig von der Prozessluft getrocknet werden. Zum Anderen passieren die Katalysatorträger-Formkörper aufgrund der durch die kontrollierte Luftgleitschicht bewirkten konsequenten Orbitalbewegung der Formkörper den Sprühvorgang (Applikation der Vorläuferverbindungen)in nahezu konstanter Häufigkeit. Dadurch wird eine weitgehend einheitliche Schalendicke einer behandelten Charge von Formkörpern erreicht. Ferner wird dadurch erreicht, dass die Edelmetallkonzentration über einen großen Bereich der Schalendicke hinweg nur gering variiert, d.h., dass die Edelmetallkonzentration über einen großen Bereich der Schalendicke hinweg eine Rechteckfunktion beschreibt, wodurch eine einheitliche Aktivität des resultierenden Katalysators über die Dicke der Pd/Au-Schale hinweg gewährleistet ist.

Geeignete Dragiertrommeln, Wirbelschichtanlagen bzw. Fließbettanlagen zur Durchführung des erfindungsgemäßen Verfahrens entsprechend bevorzugter Ausführungsformen sind im Stand der Technik bekannt und werden z.B. von den Unternehmen Heinrich Brucks GmbH (Alfeld, Deutschland), ERWEK GmbH (Heusenstamm, Deutschland), Stechel (Deutschland), DRIAM Anlagenbau GmbH (Eriskirch, Deutschland), Glatt GmbH (Binzen, Deutschland), G.S. Divisione Verniciatura (Osteria, Italien), HOFER-Pharma Maschinen GmbH (Weil am Rhein, Deutschland), L. B. Bohle Maschinen + Verfahren GmbH (Enningerloh, Deutschland), Lödige Maschinenbau GmbH (Paderborn, Deutschland), Manesty (Merseyside, Großbritannien), Vector Corporation (Marion, IA, USA), Aeromatic-Fielder AG (Bubendorf, Schweiz), GEA Process Engineering (Hampshire, Großbritannien), Fluid Air Inc. (Aurora, Illinois, USA), Heinen Systems GmbH (Varel, Deutschland), Hüttlin GmbH (Steinen, Deutschland), Umang Pharmatech Pvt. Ltd. (Marharashtra, Indien) und Innojet Technologies (Lörrach, Deutschland) vertrieben. Besonders bevorzugte Fließbettvorrichtungen werden unter der Bezeichnung Innojet® Aircoater oder Innojet® Ventilus von der Firma Innojet Technologies vertrieben.

Gemäß einer weiter bevorzugten Ausführungsform des erfindungsgemäßen Verfahrens wird der Katalysatorträger während des Auftragens der Lösungen erwärmt, beispielsweise mittels erwärmter Prozessluft. Über den Grad der Erwärmung der Katalysatorträger kann die Abtrocknungsgeschwindigkeit der aufgetragenen Lösungen der Edelmetall-Vorläuferverbindungen bestimmt werden. Bei relativ niedrigen Temperaturen ist die Abtrocknungsgeschwindigkeit beispielsweise verhältnismäßig klein, so dass es bei entsprechendem quantitativem Auftrag aufgrund der durch das Vorhandensein von Lösungsmittel bedingten hohen Diffusion der Vorläuferverbindungen zur Ausbildung größerer Schalendicken kommen kann. Bei relativ hohen Temperaturen ist die Abtrocknungsgeschwindigkeit beispielsweise verhältnismäßig hoch, so dass mit dem Formkörper in Kontakt kommende Lösung der Vorläuferverbindungen nahezu unverzüglich abtrocknet, weshalb auf dem Katalysatorträger aufgetragene Lösung nicht tief in denselben eindringen kann. Bei verhältnismäßig hohen Temperaturen können so relativ kleine Schalendicken mit hoher Edelmetallbeladung erhalten werden. Beispielsweise kann der Katalysatorträger auf eine Temperatur von 40 bis 80 °C erwärmt werden.

In den im Stand der Technik beschriebenen Verfahren zur Herstellung von VAM-Schalenkatalysatoren auf der Basis von Pd und Au werden üblicherweise kommerziell erhältliche Lösungen der Vorläuferverbindungen wie $Na_2PdCl_4$-, $NaAuCl_4$- oder $HAuCl_4$-Lösungen eingesetzt. In der jüngeren Literatur werden, wie bereits vorstehend ausgeführt, auch chloridfreie Pd- oder Au-Vorläuferverbindungen wie beispielsweise $Pd(NH_3)_4(OH)_2$, $Pd(NH_3)_2(NO_2)_2$ und $KAuO_2$ eingesetzt. Diese Vorläuferverbindungen reagieren in Lösung basisch, während die klassischen Chlorid-, Nitrat- und Acetat-Vorläuferverbindungen in Lösung sauer reagieren.

Für den Auftrag der Vorläuferverbindungen auf den Katalysatorträger werden üblicherweise bevorzugt wässrige $Na_2PdCl_4$- und $NaAuCl_3$-Lösungen verwendet. Diese Metallsalzlösungen werden normalerweise bei Raumtemperatur auf den Träger aufgebracht und anschließend die Metall-Komponenten mit NaOH als unlösliche Pd- bzw. Au-Hydroxide fixiert. Danach wird der beladene Träger üblicherweise mit Wasser chloridfrei gewaschen. Insbesondere die Au-Fixierung ist dabei mit Nachteilen behaftet, wie lange Einwirkzeiten der Base, um die Fällung des stabilen Au-Tetrachlorokomplexes zu induzieren, unvollständige Fällung und damit verbundener mangelhafter Au-Retention.

Entsprechend einer weiter bevorzugten Ausführungsform des erfindungsgemäßen Verfahrens umfasst das Verfahren die Schritte:

a) Bereitstellen einer ersten Lösung einer Pd- und/oder einer Au-Vorläuferverbindung;

b) Bereitstellen einer zweiten Lösung einer Pd- und/oder einer Au-Vorläufer-verbindung, wobei die erste Lösung eine Ausfällung der Edelmetall-Komponente/n der Vorläuferverbindung/en der zweiten Lösung bewirkt und umgekehrt;

c) Auftragen der ersten Lösung und der zweiten Lösung auf den Katalysatorträger.

Diese Ausführungsform des erfindungsgemäßen Verfahrens nutzt zwei voneinander verschiedene Vorläuferlösungen, von denen beispielsweise die eine eine Pd- und die andere eine Au-Vorläuferverbindung enthält. Dabei weist vorzugsweise in der Regel die eine der Lösungen einen basischen und die andere einen sauren pH-Wert auf. Der Auftrag der Lösungen auf den Katalysatorträger erfolgt in der Regel, indem zunächst der Träger mit der ersten und anschließend in einem darauffolgenden Schritt mit der zweiten Lösung wie vorstehend beschrieben, beispielsweise durch Tränken, imprägniert wird. Beim Auftrag der zweiten Lösung werden dann die beiden Lösungen auf dem Träger vereinigt, wodurch sich der pH-Wert der Lösungen ändert und die Pd- bzw. Au-Komponente der jeweiligen Vorläuferverbindung auf den Träger ausgefällt wird, ohne dass dazu eine wie im Stand der Technik übliche Hilfsbase wie NaOH oder KOH auf den Träger aufgebracht werden muss.

Die genannte Ausführungsform des erfindungsgemäßen Verfahrens basiert also auf einer Imprägnierung des Katalysatorträgers mit der ersten Lösung einer Pd- und/oder Au-Vorläuferverbindung und der zweiten Lösung einer Pd- und/oder Au-Vorläuferverbindung, wobei die beiden Lösungen zueinander inkompatibel sind, d.h., dass die erste Lösung eine Ausfällung der Edelmetall-Komponente/n der Vorläuferverbindung/en der zweiten Lösung bewirkt und umgekehrt, so dass in der Kontaktzone beider Lösungen sowohl die vorimprägnierte/n Pd-/Au-Komponente/n als auch die nachimprägnierte/n Pd-/Au-Komponente/n nahezu gleichzeitig ausfallen und somit zu einer innigen Pd/Au-Durchmischung führen. Zwischen den beiden Imprägnierschritten kann optional getrocknet werden.

Geeignete wässrige Lösungen von Pd-Vorläuferverbindungen für die Imprägnierung mit inkompatiblen Lösungen sind beispielhaft in der Tabelle 1 aufgeführt.

Tabelle 1:

| Vorläuferverbindung | Charakter der Lösung |
|---|---|
| $PdCl_2$ | sauer |
| $Pd(NH_3)_2(NO_2)_2$ | basisch |
| $Na_2PdCl_4$ | neutral |
| $Pd(NH_3)_4(OH)_2$ | basisch |
| $Pd(NO_3)_2$ | sauer |
| $K_2Pd(OAc)_2(OH)_2$ | basisch durch Auflösen von Palladiumacetat in KOH |

Falls $NH_3$ hinsichtlich einer vorzeitigen Au-Reduktion zu stark reduzierend wirken sollte, können anstelle der Palladiumaminkomplexe auch die entsprechenden Diaminkomplexe mit Ethylendiamin als Ligand oder die entsprechenden Ethanolaminkomplexe verwendet werden.

Geeignete wässrige Lösungen von Au-Vorläuferverbindungen für die Imprägnierung mit inkompatiblen Lösungen sind beispielhaft in der Tabelle 2 aufgeführt.

Tabelle 2:

| Vorläuferverbindung | Charakter der Lösung |
|---|---|
| $AuCl_3$ | sauer |
| $KAuO_2$ | basisch durch Auflösen von $Au(OH)_3$ in KOH |

(fortgesetzt)

| Vorläuferverbindung | Charakter der Lösung |
|---|---|
| NaAuCl$_4$ | neutral |
| HAuCl$_4$ | sauer |
| KAu(OAc)$_3$(OH) | basisch durch Auflösen von Au(OAc)$_3$ in KOH |
| HAu(NO$_3$)$_4$ | sauer (stabil in halbkonzentrierter HNO$_3$) |

Geeignete Kombinationen von inkompatiblen Lösungen zur basenfreien Fällung der Edelmetallkomponenten sind beispielsweise eine PdCl$_2$- und eine KAuO$_2$-Lösung; eine Pd(NO$_3$)$_2$- und eine KAuO2-Lösung; eine Pd(NH$_3$)$_4$(OH)$_2$- und eine AuCl$_3$- oder HAuCl$_4$-Lösung.

Gemäß einer weiter bevorzugten Ausführungsform des erfindungsgemäßen Verfahrens kann auch Pd mit inkompatiblen Pd-Lösungen gefällt werden und analog Au mit inkompatiblen Au-Lösungen, z.B. durch Inkontaktbringen einer PdCl$_2$-Lösung mit einer Pd(NH$_3$)$_4$(OH)$_2$-Lösung bzw. einer HAuCl$_4$- mit einer KAu02-Lösung. Auf diese Weise lassen sich hohe Pd- und/oder Au-Gehalte in der Schale abscheiden, ohne hochkonzentrierte Lösungen einsetzen zu müssen.

Gemäß einer weiteren Ausführungsform des erfindungsgemäßen Verfahrens können auch miteinander kompatible Mischlösungen eingesetzt werden, die zur Edelmetall-Fällung mit einer zu der Mischlösung inkompatiblen Lösung in Kontakt gebracht werden. Ein Beispiel für eine Mischlösung ist eine PdCl$_2$ und AuCl$_3$ enthaltende Lösung, deren Edelmetall-Komponenten mit einer KAuO$_2$-Lösung gefällt werden können, oder eine Pd(NH$_3$)$_4$(OH)$_2$ und KAuO$_2$ enthaltende Lösung, deren Edelmetall-Komponenten mit einer PdCl$_2$ und HAuCl$_4$ enthaltenden Lösung gefällt werden können. Ein weiteres Beispiel für eine Mischlösung ist das Paar HAuCl$_4$ und KAuO$_2$.

Die Imprägnierung mit den inkompatiblen Lösungen wird vorzugsweise mittels Tränken oder mittels Sprühimprägnierungen erfolgen, wobei die inkompatiblen Lösungen beispielsweise simultan durch eine oder mehrere Doppeldüse(n) oder simultan mittels zweier Düsen oder Düsengruppen oder sequentiell mittels einer oder mehreren Düse(n) versprüht werden.

Die Imprägnierung mit den inkompatiblen Lösungen kann aufgrund der schnellen Immobilisierung (Fixierung) der Metall-Komponenten der Vorläuferverbindungen in der Schale und der damit einhergehenden verkürzten Pd- und Au-Diffusion zu dünneren Schalen führen als die herkömmliche Verwendung von miteinander kompatiblen Lösungen. Mittels der inkompatiblen Lösungen können hohe Edelmetall-Gehalte in dünnen Schalen, verbesserte Metallretention, schnellere und vollständigere Fällung der Edelmetalle, die Verminderung des störenden Na-Restgehalts des Trägers, die gleichzeitige Fixierung von Pd und Au in nur einem Fixierschritt sowie der Wegfall der NaOH-Kosten und der NaOH-Handhabung und eine Vermeidung einer mechanischen Schwächung des Trägers durch den Kontakt mit überschüssiger NaOH erreicht werden.

Mittels der Imprägnierung mit inkompatiblen Lösungen können durch einen einzigen Fixierschritt, der lediglich den Auftrag zweier inkompatibler Lösungen beinhaltet, größere Edelmetall-Gehalte auf dem Katalysatorträger abgeschieden werden als dies mit der klassischen Basen(NaOH)-Fixierung möglich ist. Insbesondere lassen sich mittels des Prinzips der inkompatiblen Lösungen hohe Au-Gehalte mit einem Au/Pd-Atomverhältnis von 0,6 und mehr leicht erreichen, was hinsichtlich der Erhöhung der VAM-Selektivität sehr erwünscht ist.

Entsprechend einer weiter bevorzugten Ausführungsform des erfindungsgemäßen Verfahrens ist vorgesehen, dass der Katalysatorträger, nachdem die Pd- und/oder die Au-Vorläuferverbindung auf den Katalysatorträger aufgetragen worden ist/sind zur Fixierung der Edelmetall-Komponente/n der Vorläuferverbindung/en auf dem Katalysatorträger einem Fixierungsschritt unterworfen wird. Der Fixierungsschritt kann dabei die Behandlung des Trägers mit Lauge oder Säure beinhalten, je nachdem, ob die Vorläuferverbindung sauer bzw. basisch ist, oder eine Kalzinierung des Trägers zur Überführung der Edelmetall-Komponente/n in eine HydroxidVerbindung/en bzw. in ein Oxid vorgesehen ist. Der Fixierschritt kann auch ausgelassen werden und die Edelmetall-Komponenten direkt reduziert werden, z.B. durch die Behandlung mit einer reduzierend wirkenden Gasphase, z.B. Ethylen, etc. bei erhöhten Temperaturen von 20 °C bis 200 °C.

a) Nach dem Beladen mit den Vorläuferverbindungen bzw. nach der Fixierung der Edelmetall-Komponenten kann der Träger zur Überführung der Edelmetall-Komponenten in die entsprechenden Oxide kalziniert werden. Die Kalzinierung erfolgt bevorzugt bei Temperaturen von weniger als 700 °C. Besonders bevorzugt zwischen 300-450 °C unter Luftzutritt. Die Kalzinierdauer ist abhängig von der Kalziniertemperatur und wird bevorzugt im Bereich von 0,5-6 Stunden gewählt. Bei einer Kalziniertemperatur von etwa 400 °C beträgt die Kalzinierdauer bevorzugt 1-2 Stunden. Bei einer Kalziniertemperatur von 300 °C beträgt die Kalzinierdauer bevorzugt bis zu 6 Stunden.

Die Edelmetall-Komponenten werden vor dem Einsatz des Katalysators noch reduziert, wobei die Reduktion in situ, d.h. im Prozessreaktor, oder auch ex situ, d.h. in einem speziellen Reduktionsreaktor, durchgeführt werden kann. Die Reduktion in situ wird vorzugsweise mit Ethylen (5 Vol.-%) in Stickstoff bei einer Temperatur von etwa 150 °C über einen Zeitraum von beispielsweise 5 Stunden durchgeführt. Die Reduktion ex situ kann beispielsweise mit 5 Vol.-% Wasserstoff in Stickstoff, beispielsweise mittels Formiergas, bei Temperaturen im Bereich von vorzugsweise 150-500 °C über einen Zeitraum von 5 Stunden durchgeführt werden.

Gasförmige oder verdampfbare Reduktionsmittel wie beispielsweise CO, $NH_3$, Formaldehyd, Methanol und Kohlenwasserstoffe können ebenfalls eingesetzt werden, wobei die gasförmigen Reduktionsmittel auch mit Inertgas, wie beispielsweise Kohlendioxid, Stickstoff oder Argon, verdünnt sein können. Vorzugsweise wird ein Inertgas-verdünntes Reduktionsmittel eingesetzt. Bevorzugt sind Mischungen von Wasserstoff mit Stickstoff oder Argon, vorzugsweise mit einem Wasserstoffgehalt zwischen 1 Vol.-% und 15 Vol.-%.

Die Reduktion der Edelmetalle kann auch in flüssiger Phase vorgenommen werden, vorzugsweise mittels der Reduktionsmittel Hydrazin, K-Formiat, $H_2O_2$, Na-Hypophosphit, Na-Formiat, Ammoniumformiat, Ameisensäure, K-Hypophosphit oder hypophosphorige Säure.

Die Menge an Reduktionsmittel wird vorzugsweise so gewählt, dass während der Behandlungsdauer zumindest das zur vollständigen Reduktion der Edelmetall-Komponenten nötige Äquivalent über den Katalysator geleitet wird. Bevorzugt wird jedoch ein Überschuss an Reduktionsmittel über den Katalysator geleitet, um eine schnelle und vollständige Reduktion zu gewährleisten.

Vorzugsweise wird drucklos, d.h. bei einem Absolutdruck von ca. 1 bar, reduziert. Für die Herstellung technischer Mengen an erfindungsgemäßem Katalysator wird bevorzugt ein Drehrohrofen oder Wirbelschichtreaktor verwendet, um eine gleichmäßige Reduktion des Katalysators zu gewährleisten.

Die Erfindung betrifft ferner die Verwendung des erfindungsgemäßen Katalysators als Katalysator in der Synthese von Alkenylalkanoaten, insbesondere in der Synthese von Vinylacetat-Monomer (VAM), insbesondere in der Gasphasenoxidation von Ethylen und Essigsäure zu Vinylacetat-Monomer.

Bevorzugt wird der erfindungsgemäße Katalysator zur Herstellung von VAM verwendet. Diese erfolgt im Allgemeinen durch Leiten von Essigsäure, Ethylen und Sauerstoff oder Sauerstoff enthaltenden Gasen bei Temperaturen von 100-200 °C, vorzugsweise 120-200 °C, und bei Drücken von 1-25 bar, vorzugsweise 1-20 bar, über den erfindungsgemäßen Katalysator, wobei nicht umgesetzte Edukte im Kreis geführt werden können.

Zweckmäßig hält man die Sauerstoffkonzentration unter 10 Vol.-%. Unter Umständen ist jedoch auch eine Verdünnung mit inerten Gasen wie Stickstoff oder Kohlendioxid vorteilhaft. Besonders Kohlendioxid eignet sich zur Verdünnung, da es im Zuge der VAM-Synthese in geringen Mengen gebildet wird. Das entstandene Vinylacetat wird mit Hilfe geeigneter Methoden isoliert, die beispielsweise in der US 5,066,365 A beschrieben sind.

Die nachfolgenden Ausführungsbeispiele dienen in Zusammenschau mit einem Vergleichsbeispiel der Erläuterung der Erfindung:

Beispiel 1:

500 g verschiedener säurebehandelter getrockneter pulverförmiger Bentonitgemische als Schichtsilikat basierend auf natürlichen Bentoniten mit Hauptbestandteil Montmorillonit wurden mit bis zu 50 g handelsüblichem $ZrO_2$ und 12 g Methylcellulose mittels einer Kugelmühle zu einem innigen Gemisch vermahlen.

Das resultierende Gemisch wurde mit Wasser aufgenommen und mittels eines Mischers zu einem Teig verarbeitet, aus dem mittels einer Tablettenpresse unter Druck kugelförmige Formkörper hergestellt wurden. Zur Aushärtung wurden die Kugeln bei einer Temperatur von 600 °C über einen Zeitraum von 5 h kalziniert. Die so erhältlichen Formkörper weisen die in der Tabelle 3 aufgeführten Charakteristika auf:

Tabelle 3:

| Geometrische Form | Kugel |
|---|---|
| Durchmesser | 5 mm |
| Feuchtegehalt | <2,0 Mass.-% |
| Druckfestigkeit | > 35 N |
| Schüttgewicht | 550 - 600 $gl^{-1}$ |
| Wassersaugvermögen | 55 - 70 % |
| spezif. Oberfläche (BET) | 120-160 $m^2\ g^{-1}$ |
| $SiO_2$-Gehalt | 80 bis 90 Mass.-% |

(fortgesetzt)

| Geometrische Form | Kugel |
|---|---|
| $ZrO_2$-Gehalt | 3,7 bis 9,5 Mass.-% |
| Andere Oxide | Restmasse in Mass.-% |
| Glühverlust 1000 °C | <0,4 Mass.-% |
| Azidität | 10 bis 100 $\mu$val/g |
| BJH Porenvolumen $N_2$ | 0,3 - 0,45 $cm^3$ $g^{-1}$ |

225 g der wie vorstehend hergestellten Kugeln wurden in einer Fließbettvorrichtung des Unternehmens Innojet Technologies (Lörrach, Deutschland) mit der Handelsbezeichnung Innojet® Aircoater gefüllt und mittels auf 80 °C temperierter Druckluft (6 bar) in einen Fließbettzustand versetzt, in welchem die Formkörper toroidal umliefen, d.h. sich auf einer vertikal ausgerichteten ellipsoiden und einer dazu senkrecht ausgerichteten horizontalen kreisförmigen Bahn bewegten.

Nachdem die Formkörper auf eine Temperatur von ca. 75 °C temperiert waren, wurden auf das Fließbett der Formkörper 300 ml einer wässrigen Edelmetallmischlösung enthaltend 7,5 g handelsübliches $Na_2PdCl_4$ (Natriumtetrachloropalladat) und 4,6 g handelsübliches $NaAuCl_4$ (Natriumtetrachloroaurat) über einen Zeitraum von 40 min aufgesprüht.

Nach der Imprägnierung der Katalysatorträger mit der Edelmetallmischlösung wurden die Trägerkugel im Fließbettzustand unter den obigen Bedingungen über einen Zeitraum von 30 min mit einer 0,05 molaren NaOH-Lösung besprüht. Das NaOH ließ man 16 h auf die Formkörper einwirken.

Nach der NaOH-Einwirkung wurden die Träger ausgiebig in der Fließbettvorrichtung mit Wasser gewaschen, um den Träger weitestgehend von über die Edelmetallverbindungen und NaOH in den Träger eingebrachten Alkalimetall und Chlorid zu befreien.

Nach dem Waschen wurden die Formkörper in der Fließbettvorrichtung bei einer Temperatur von 90 bis 110 °C getrocknet.

Nach der Trocknung der Formkörper wurden diese mit einem Gasgemisch von Ethylen (5 Vol.-%) in Stickstoff bei einer Temperatur von etwa 150 °C in der Fließbettvorrichtung zu einem Pd/Au-Schalenkatalysator reduziert.

Der resultierende Schalenkatalysator enthielt ca. 1,2 Mass.-% Pd und wies ein Au/Pd-Atomverhältnis von ca. 0,5 (mittels ICP (inductively coupled plasma) bestimmt), eine Schalendicke von ca. 180 $\mu$m sowie eine Härte von 33 N auf.

Die Edelmetallkonzentration des so hergestellten Pd/Au-Schalenkatalysators wich über einen Bereich von 90 % der Schalendicke hinweg, wobei der Bereich zur äußeren und inneren Schalengrenze jeweils um 5 % der Schalendicke beabstandet ist, von der mittleren Edelmetallkonzentration dieses Bereichs um maximal +/- 10 % ab. Die Bestimmung der Edelmetallverteilung erfolgte an einem Rasterelektronenmikroskop LEO 430VP, ausgerüstet mit einem energiedispersiven Spektrometer der Fa. Bruker AXS. Zur Messung der Edelmetallkonzentration über die Schalendicke hinweg wurde eine Katalysatorkugel durchschnitten, auf einen Aluminiumprobenhalter geklebt und anschließend mit Kohlenstoff bedampft. Als Detektor kam ein stickstofffreier Siliziumdriftkammerdetektor (XFlash® 410) mit einer Energieauflösung von 125 eV für die Mangan $K_{alpha}$-Linie zum Einsatz.

Beispiel 2:

65 g Träger, hergestellt gemäß Beispiel 1 mit nachgeschalteter Säurebehandlung mit den in Tabelle 4 aufgelisteten Charakteristika:

Tabelle 4:

| Geometrische Form | Kugel |
|---|---|
| Durchmesser | 5 mm |
| Feuchtegehalt | <2,0 Mass.-% |
| Druckfestigkeit | 50 N |
| Schüttgewicht | 585 $gl^{-1}$ |
| Wassersaugvermögen | 61,3 % |
| spezif. Oberfläche (BET) | 158 $m^2$ $g^{-1}$ |

EP 2 158 035 B1

(fortgesetzt)

| Geometrische Form | Kugel |
|---|---|
| $SiO_2$-Gehalt | 83,3 Mass.-% |
| $ZrO_2$-Gehalt | 5,2 Mass.-% |
| Andere Oxide | Restmasse in Mass.-% |
| Glühverlust 1000 °C | 3,6 Mass.-% |
| Azidität | 30 $\mu$val/g |
| BJH Porenvolumen $N_2$ | 0,389 $cm^3$ $g^{-1}$ |

wurden gemäß dem Porenfüllverfahren (incipient-wetness-Methode), bei welchem ein Träger mit einem seinem Porenvolumen entsprechenden Lösungsvolumen imprägniert wird, mit 38,6 ml einer wässrigen Lösung beinhaltend 1,568 g $Na_2PdCl_4$ und 0,357 g $HAuCl_4$ imprägniert. Nach der Imprägnierung wurden 89,14 g einer 0,35 molaren NaOH-Lösung auf die Katalysatorträger gegeben und über Nacht bei Raumtemperatur für 21 h stehen gelassen. Nach Dekantieren der Fixierlösung wurde der so hergestellte Katalysatorvorläufer mit 73,65 g einer 10 %-igen $NaH_2PO_2$-Lösung 2 h lang reduziert. Nach Ablassen der Reduktionslösung wurden die Katalysatoren mit dest. Wasser 8 h bei Raumtemperatur unter ständigem Austausch des Wassers (Durchfluss = 140 rpm) zur Entfernung von Cl-Resten gewaschen. Der Endwert der Leitfähigkeit der Waschlösung betrug 1,4 $\mu$S.

Im Anschluss wurde der Katalysator in der Wirbelschicht bei 90 °C für 70 min getrocknet. Die getrockneten Kugeln wurden mit einer Mischung aus 27,30 g 2 molarer KOAc-Lösung und 13,59 g $H_2O$ beladen und 1 Stunde bei Raumtemperatur stehen gelassen. Zum Abschluss erfolgt die Trocknung für 70 min bei 90 °C in der Wirbelschicht.

Die theoretische Beladung der Träger mit Pd beträgt 0,8 Gew.-% und 0,3 Gew.-% Au; die experimentelle Beladung betrug 0,78 Gew.-% Pd und 0,27 Gew.-% Au (mittels ICP bestimmt).

Die Schalendicke betrug 239 $\mu$m (statistischer Mittelwert).

6 ml einer Schüttung von Katalysatorkugeln des Beispiels 2 wurden in einem Festbettröhrenreaktor bei einer Temperatur von 150 °C bei 10 bar mit einem Feedgasstrom von 550 Nml/min zusammengesetzt aus 15 % HOAc, 6 % $O_2$, 39 % $C_2H_4$ in $N_2$ beaufschlagt und der Reaktoraustrag mittels Gaschromatographie analysiert.

Die Selektivität (von Ethylen zu VAM) wird nach der Formel $S(C_2H_4)$ = mole VAM / (mole VAM + mole $CO_2$/2) berechnet. Die Raum-Zeit-Ausbeute ergibt sich als g VAM/l Katalysator/h. Der Sauerstoff-Umsatz wird berechnet nach (mole $O_2$ in - mole $O_2$ out)/mole $O_2$ in.

Der mittels des erfindungsgemäßen Katalysatorträgers hergestellte erfindungsgemäße Katalysator gemäß Beispiel 2 zeigt eine Selektivität $S(C_2H_4)$ von 92,4 % sowie eine Raum-Zeit-Ausbeute (gaschromatographisch bestimmt) von 840 g VAM / l Katalysator / h bei einem Sauerstoffumsatz von 49,5 %.

Vergleichsbeispiel:

100 g eines Bentonit-haltigen Trägers der Firma SÜD-Chemie AG (München, Deutschland) mit der Handelsbezeichnung "KA-160" mit den in der Tabelle 5 aufgeführten Charakteristika:

Tabelle 5:

| Geometrische Form | Kugel |
|---|---|
| Durchmesser | 5 mm |
| Feuchtegehalt | <2,0 Mass.-% |
| Druckfestigkeit | > 60 N |
| Schüttgewicht | 554 g $l^{-1}$ |
| Wassersaugvermögen | 62 % |
| spezif. Oberfläche (BET) | 158 $m^2$ $g^{-1}$ |
| $SiO_2$-Gehalt | 93,2 Mass.-% |
| $Al_2O_3$-Gehalt | 2,2 Mass.-% |
| $Fe_2O_3$-Gehalt | 0,35 Mass.-% |

(fortgesetzt)

| Geometrische Form | Kugel |
|---|---|
| $TiO_2$-Gehalt | (Summe) < 1,5 Mass.-% |
| MgO-Gehalt | |
| CaO-Gehalt | |
| $K_2O$-Gehalt | |
| $Na_2O$-Gehalt | |
| Glühverlust 1000 °C | <0,3 Mass.-% |
| Azidität | 53 $\mu$val/g |
| BJH Porenvolumen $N_2$ | 0,38 $cm^3 \, g^{-1}$ |

wurden mit 62 ml einer 9,1 %-igen (bezogen auf Zr), wässrigen Zirkonylnitratlösung entsprechend dem Porenfüllverfahren (incipient wetness-Methode) imprägniert. Anschließend wurde der Träger bei 120 °C über einen Zeitraum von 5 h getrocknet und danach bei 450 °C an der Luft für 2 h kalziniert. Auf diese Weise wurde ein mit 5,2 Gew.-% $ZrO_2$ oberflächendotierter KA 160 Träger erhalten.

Unter Verwendung des oberflächendotierten KA 160 Trägers wurde analog Beispiel 2 ein Katalysator hergestellt.

Die experimentelle Beladung der Träger mit Pd betrug 0,77 Gew.-%, die mit Au 0,27 Gew.-% (mittels ICP bestimmt).

Die Schalendicke betrug 260 $\mu$m (statistischer Mittelwert).

Die Performance des Katalysators des Vergleichsbeispiels wurde analog Beispiel 2 bestimmt und zeigt eine Selektivität $S(C_2H_4)$ von 92,1 % sowie eine Raum-Zeit-Ausbeute (gaschromatographisch bestimmt) von 410 g VAM / l Katalysator / h bei einem Sauerstoffumsatz von 24,5 %.

**Patentansprüche**

1. Poröser Katalysatorträger, bestehend aus einem ein natürliches Schichtsilikat umfassendes Material, wobei $ZrO_2$ in dem Material verteilt enthalten ist, **dadurch gekennzeichnet, dass** es sich bei dem natürlichen Schichtsilikat um einen säurebehandelten, kalzinierten Bentonit handelt, wobei das $ZrO_2$ in dem Material gleichmäßig verteilt enthalten ist und das $ZrO_2$ in partikulärer Form vorliegt, der Katalysatorträger als Kugel ausgebildet ist und eine Härte von größer/gleich 20 N aufweist, wobei die Härte wie in der Beschreibung ausgeführt bestimmt wird.

2. Katalysatorträger nach Anspruch 1, **dadurch gekennzeichnet, dass** die Zirkoniumoxidpartikel in dem Katalysatorträger mit einem Anteil von 1 bis 25 Mass.-% enthalten sind, vorzugsweise mit einem Anteil von 3 bis 20 Mass.-% und bevorzugt mit einem Anteil von 5 bis 20 Mass.-% bezogen auf die Masse des Katalysatorträgers.

3. Katalysatorträger nach einem der Ansprüche 1 oder 2, **dadurch gekennzeichnet, dass** der Anteil des Katalysatorträgers an säurebehandeltem kalziniertem Bentonit größer/gleich 50 Mass.-% ist, vorzugsweise größer/gleich 60 Mass.-%, bevorzugt größer/gleich 70 Mass.-%, weiter bevorzugt größer/gleich 80 Mass.-%, mehr bevorzugt größer/gleich 90 Mass.-% und am meisten bevorzugt größer/gleich 93 Mass.-% bezogen auf die Masse des Katalysatorträgers.

4. Katalysatorträger nach einem der Ansprüche 1 bis 3, **dadurch gekennzeichnet, dass** der im Träger enthaltene säurebehandelte, kalzinierte Bentonit einen $SiO_2$-Gehalt von zumindest 65 Mass.-% aufweist, vorzugsweise einen von zumindest 80 Mass.-% und bevorzugt einen von 95 bis 99,5 Mass.-% und dass der im Träger enthaltene säurebehandelte, kalzinierte Bentonit weniger als 10 Mass.-% $Al_2O_3$ enthält, vorzugsweise 0,1 bis 3 Mass.-% und bevorzugt 0,3 bis 1,0 Mass.-%.

5. Katalysatorträger nach einem der Ansprüche 1 bis 4, **dadurch gekennzeichnet, dass** der Katalysatorträger mit zumindest einem Oxid eines Metalls dotiert ist, ausgewählt aus der Gruppe bestehend aus Hf, Ti, Nb, Ta, W, Mg, Re, Y und Fe, vorzugsweise mit $HfO_2$ oder $Fe_2O_3$ und wobei der Anteil des Katalysatorträgers an Dotierungsoxid zwischen 0 und 20 Mass.-% beträgt, vorzugsweise 1,0 bis 10 Mass.-% und bevorzugt 3 bis 8 Mass.-%.

**6.** Verfahren zur Herstellung eines Katalysatorträgers nach einem der Ansprüche 1 bis 5, umfassend die folgenden Schritte:

a) Vermischen eines pulverförmigen, säurebehandelten Bentonits, mit pulverförmigem Zirkonmetall oder einer pulverförmigen Zirkoniumverbindung;
b) Kalzinieren des erhaltenen Gemisches beziehungsweise des Formkörpers bei einer Temperatur von 400 bis 800°C;
c) Formen eines Formkörpers aus dem erhaltenen Gemisch, vorzugsweise vor Schritt b).

**7.** Verfahren nach Anspruch 6, **dadurch gekennzeichnet, dass** das Verfahren ferner den Schritt umfasst: Behandeln des kalzinierten Gemisches mit einer Säure.

**8.** Verfahren nach einem der Ansprüche 6 oder 7, **dadurch gekennzeichnet, dass** in Schritt a) desweiteren $Y_2O_3$ und/oder $HfO_2$ eingesetzt wird.

**9.** Schalenkatalysator für die Herstellung von Vinylacetat-Monomer (VAM), umfassend einen Katalysatorträger nach einem der Ansprüche 1 bis 5, in dessen äußerer Schale metallisches Pd und Au enthalten ist.

**10.** Schalenkatalysator nach Anspruch 9, **dadurch gekennzeichnet, dass** die Schale des Katalysators eine Dicke von kleiner als 300 $\mu$m aufweist, vorzugsweise eine von kleiner als 200 $\mu$m, bevorzugt eine von kleiner als 150 $\mu$m, weiter bevorzugt eine von kleiner als 100 $\mu$m und mehr bevorzugt eine von kleiner als 80 $\mu$m.

**11.** Schalenkatalysator nach einem der Ansprüche 9 oder 10, **dadurch gekennzeichnet, dass** der Anteil des Katalysators an Pd 0,5 bis 2,5 Mass.-% beträgt, vorzugsweise 0,6 bis 2,3 Mass.-% und bevorzugt 0,7 bis 2 Mass.-% bezogen auf die Masse des mit Edelmetall beladenen Katalysatorträgers und wobei das Au/Pd-Atomverhältnis des Katalysators zwischen 0 und 1,2 liegt, vorzugsweise zwischen 0,1 und 1, bevorzugt zwischen 0,3 und 0,9 und besonders bevorzugt zwischen 0,4 und 0,8.

**12.** Schalenkatalysator nach einem der Ansprüche 9 bis 11, **dadurch gekennzeichnet, dass** der Katalysator ein Alkalimetallacetat umfasst, vorzugsweise Kaliumacetat, wobei das Alkalimetall/ Pd-Atomverhältnis zwischen 1 und 12 beträgt, vorzugsweise zwischen 2 und 10 und bevorzugt zwischen 4 und 9.

**13.** Verfahren zur Herstellung eines Schalenkatalysators nach einem der Ansprüche 9 bis 12, umfassend die Schritte

a) Bereitstellen eines Katalysatorträgers nach einem der Ansprüche 1 bis 5;
b) Auftragen einer Lösung einer Pd-Vorläuferverbindung auf den Katalysatorträger;
c) Auftragen einer Lösung einer Au-Vorläuferverbindung auf den Katalysatorträger;
d) Überführen der Pd-Komponente der Pd-Vorläuferverbindung in die metallische Form; und
e) Überführen der Au-Komponente der Au-Vorläuferverbindung in die metallische Form.

**14.** Verfahren nach Anspruch 13, **dadurch gekennzeichnet, dass**

a) eine erste Lösung einer Pd- und/oder einer Au-Vorläuferverbindung bereitgestellt wird;
b) eine zweite Lösung einer Pd- und/oder einer Au-Vorläufer-verbindung bereitgestellt wird, wobei die erste Lösung eine Ausfällung der Edelmetall-Komponente/n der Vorläuferverbindung/en der zweiten Lösung bewirkt und umgekehrt;
c) die erste und die zweite Lösung auf den Katalysatorträger aufgetragen wird.

**15.** Verfahren nach Anspruch 14, **dadurch gekennzeichnet**, das die Vorläuferverbindung der ersten Lösung sauer und die der zweiten Lösung basisch ist.

**16.** Verwendung eines Katalysators nach einem der Ansprüche 9 bis 12 als Katalysator in der Synthese von Alkenylalkanoaten, insbesondere in der Synthese von Vinylacetat-Monomer, insbesondere in der Gasphasenoxidation von Ethylen und Essigsäure zu Vinylacetat-Monomer.

**Claims**

1.  Porous catalyst support consisting of a material comprising a natural sheet silicate, with $ZrO_2$ being present in dispersed form in the material, **characterized in that** the natural sheet silicate is an acid-treated, calcined bentonite, with the $ZrO_2$ being uniformly distributed in the material and the $ZrO_2$ being present in particulate form, the catalyst support being configured as a sphere and having a hardness of greater than/equal to 20 N, where the hardness is determined as set forth in the description.

2.  Catalyst support according to Claim 1, **characterized in that** the zirconium oxide particles are present in the catalyst support in a proportion of from 1 to 25% by mass, preferably in a proportion of from 3 to 20% by mass and especially preferably in a proportion of from 5 to 20% by mass, based on the mass of the catalyst support.

3.  Catalyst support according to either Claim 1 or 2, **characterized in that** the proportion of acid-treated calcined bentonite in the catalyst support is greater than/equal to 50% by mass, preferably greater than/equal to 60% by mass, especially preferably greater than/equal to 70% by mass, more preferably greater than/equal to 80% by mass, even more preferably greater than/equal to 90% by mass and most preferably greater than/equal to 93% by mass, based on the mass of the catalyst support.

4.  Catalyst support according to any of Claims 1 to 3, **characterized in that** the acid-treated, calcined bentonite present in the support has an $SiO_2$ content of at least 65% by mass, preferably at least 80% by mass and especially preferably from 95 to 99.5% by mass, and **in that** the acid-treated, calcined bentonite present in the support contains less than 10% by mass of $Al_2O_3$, preferably from 0.1 to 3% by mass and especially preferably from 0.3 to 1.0% by mass.

5.  Catalyst support according to any of Claims 1 to 4, **characterized in that** the catalyst support is doped with at least one oxide of a metal selected from the group consisting of Hf, Ti, Nb, Ta, W, Mg, Re, Y and Fe, preferably with $HfO_2$ or $Fe_2O_3$, and the proportion of doping oxide in the catalyst support is between 0 and 20% by mass, preferably 1.0 to 10% by mass and especially preferably 3 to 8% by mass.

6.  Process for producing a catalyst support according to any of Claims 1 to 5, comprising the following steps:

    a) mixing a pulverulent, acid-treated bentonite with pulverulent zirconium metal or a pulverulent zirconium compound;
    b) calcining the mixture obtained or the shaped body at a temperature of from 400 to 800°C;
    c) shaping a shaped body from the mixture obtained, preferably before step b).

7.  Process according to Claim 6, **characterized in that** the process additionally comprises the step: treating the calcined mixture with an acid.

8.  Process according to either Claim 6 or 7, **characterized in that** $Y_2O_3$ and/or $HfO_2$ is additionally added in step a).

9.  Shell catalyst for preparing vinyl acetate monomer (VAM), comprising a catalyst support according to any of Claims 1 to 5 in the outer shell of which metallic Pd and Au is present.

10. Shell catalyst according to Claim 9, **characterized in that** the shell of the catalyst has a thickness of less than 300 $\mu$m, preferably less than 200 $\mu$m, especially preferably less than 150 $\mu$m, more preferably less than 100 $\mu$m and even more preferably less than 80 $\mu$m.

11. Shell catalyst according to either Claim 9 or 10, **characterized in that** the proportion of Pd in the catalyst is from 0.5 to 2.5% by mass, preferably from 0.6 to 2.3% by mass and especially preferably from 0.7 to 2% by mass, based on the mass of the catalyst support loaded with noble metal, and the Au/Pd atomic ratio of the catalyst is between 0 and 1.2, preferably between 0.1 and 1, especially preferably between 0.3 and 0.9 and particularly preferably between 0.4 and 0.8.

12. Shell catalyst according to any of Claims 9 to 11, **characterized in that** the catalyst comprises an alkali metal acetate, preferably potassium acetate, with the alkali metal/Pd atomic ratio being between 1 and 12, preferably between 2 and 10 and especially preferably between 4 and 9.

13. Process for producing a shell catalyst according to any of Claims 9 to 12, comprising the steps

a) providing a catalyst support according to any of Claims 1 to 5,
b) applying a solution of a Pd precursor compound to the catalyst support;
c) applying a solution of an Au precursor compound to the catalyst support;
d) converting the Pd component of the Pd precursor compound into the metallic form; and
e) converting the Au component of the Au precursor compound into the metallic form.

14. Process according to Claim 13, **characterized in that**

a) a first solution of a Pd precursor compound and/or an Au precursor compound is provided;
b) a second solution of a Pd precursor compound and/or an Au precursor compound is provided, where the first solution brings about precipitation of the noble metal component(s) of the precursor compound(s) of the second solution and vice versa;
c) the first solution and the second solution are applied to the catalyst support.

15. Process according to Claim 14, **characterized in that** the precursor compound of the first solution is acidic and that of the second solution is basic.

16. Use of a catalyst according to any of Claims 9 to 12 as catalyst in the synthesis of alkenyl alkanoates, in particular in the synthesis of vinyl acetate monomer, in particular in the gas-phase oxidation of ethylene and acetic acid to give vinyl acetate monomer.

## Revendications

1. Support poreux de catalyseur, constitué d'un matériau comprenant un phyllosilicate naturel, du $ZrO_2$ étant contenu sous forme répartie dans le matériau, **caractérisé en ce que** le phyllosilicate naturel est une bentonite calcinée, traitée à l'acide, le $ZrO_2$ étant contenu sous forme répartie de manière uniforme dans le matériau et le $ZrO_2$ étant présent sous forme particulaire, le support de catalyseur étant formé en tant que bille et présentant une dureté supérieure ou égale à 20 N, la dureté étant déterminée comme décrit dans la description.

2. Support de catalyseur selon la revendication 1, **caractérisé en ce que** les particules d'oxyde de zirconium sont contenues dans le support de catalyseur à raison d'une proportion de 1 à 25 % en masse, de préférence à raison d'une proportion de 3 à 20 % en masse et préférablement à raison d'une proportion de 5 à 20 % en masse par rapport à la masse du support de catalyseur.

3. Support de catalyseur selon l'une quelconque des revendications 1 et 2, **caractérisé en ce que** la proportion du support de catalyseur en bentonite calcinée traitée à l'acide est supérieure ou égale à 50 % en masse, de préférence supérieure ou égale à 60 % en masse, préférablement supérieure ou égale à 70 % en masse, encore préférablement supérieure ou égale à 80 % en masse, plus préférablement supérieure ou égale à 90 % en masse et le plus préférablement supérieure ou égale à 93 % en masse, par rapport à la masse du support de catalyseur.

4. Support de catalyseur selon l'une quelconque des revendications 1 à 3, **caractérisé en ce que** la bentonite calcinée traitée à l'acide contenue dans le support présente une teneur en $SiO_2$ d'au moins 65 % en masse, de préférence d'au moins 80 % en masse et préférablement de 95 à 99,5 % en masse et que la bentonite calcinée traitée à l'acide contenue dans le support contient moins de 10 % en masse de $Al_2O_3$, de préférence 0,1 à 3 % en masse et préférablement 0,3 à 1,0 % en masse.

5. Support de catalyseur selon l'une quelconque des revendications 1 à 4, **caractérisé en ce que** le support de catalyseur est dopé par au moins un oxyde de métal, choisi dans le groupe constitué par Hf, Ti, Nb, Ta, W, Mg, Re, Y et Fe, de préférence par $HfO_2$ ou $Fe_2O_3$ et la proportion du support de catalyseur en oxyde dopant étant comprise entre 0 et 20 % en masse, de préférence valant 1,0 à 10 % en masse et préférablement 3 à 8 % en masse.

6. Procédé pour la préparation d'un support de catalyseur selon l'une quelconque des revendications 1 à 5, comprenant les étapes suivantes :

a) mélange d'une bentonite traitée à l'acide, sous forme de poudre, avec du zirconium métal sous forme de poudre ou avec un composé de zirconium sous forme de poudre ;

b) calcination du mélange obtenu ou, selon le cas, du corps moulé, à une température de 400 à 800 °C ;
c) mise en forme d'un corps moulé à partir du mélange obtenu, de préférence avant l'étape b).

7. Procédé selon la revendication 6, **caractérisé en ce que** le procédé comprend en outre l'étape de : traitement du mélange calciné avec un acide.

8. Procédé selon l'une quelconque des revendications 6 et 7, **caractérisé en ce que** dans l'étape a), $Y_2O_3$ et/ou $HfO_2$ est/sont en outre utilisé(s).

9. Catalyseur en forme de coquille pour la préparation de monomères d'acétate de vinyle (MAV), comprenant un support de catalyseur selon l'une quelconque des revendications 1 à 5, dans lequel les métaux Pd et Au sont contenus dans l'enveloppe extérieure.

10. Catalyseur en forme de coquille selon la revendication 9, **caractérisé en ce que** l'enveloppe du catalyseur présente une épaisseur inférieure à 300 $\mu$m, de préférence inférieure à 200 $\mu$m, préférablement inférieure à 150 $\mu$m, encore préférablement inférieure à 100 $\mu$m et plus préférablement inférieure à 80 $\mu$m.

11. Catalyseur en forme de coquille selon l'une quelconque des revendications 9 et 10, **caractérisé en ce que** la proportion du catalyseur en Pd est de 0,5 à 2,5 % en masse, de préférence 0,6 à 2,3 % en masse et préférablement de 0,7 à 2 % en masse par rapport à la masse du support de catalyseur chargé par un métal noble et le rapport atomique Au/Pd du catalyseur se situant entre 0 et 1,2, de préférence entre 0,1 et 1, préférablement entre 0,3 et 0,9 et particulièrement préférablement entre 0,4 et 0,8.

12. Catalyseur en forme de coquille selon l'une quelconque des revendications 9 à 11, **caractérisé en ce que** le catalyseur comprend un acétate de métal alcalin, de préférence de l'acétate de potassium, le rapport atomique métal alcalin/Pd étant compris entre 1 et 12, de préférence entre 2 et 10 et préférablement entre 4 et 9.

13. Procédé pour la préparation d'un catalyseur en forme de coquille selon l'une quelconque des revendications 9 à 12, comprenant les étapes

a) mise à disposition d'un support de catalyseur selon l'une quelconque des revendications 1 à 5 ;
b) application d'une solution d'un composé de précurseur de Pd sur le support de catalyseur ;
c) application d'une solution d'un composé de précurseur d'Au sur le support de catalyseur ;
d) conversion sous forme métallique du composant de Pd du composé de précurseur de Pd ;
et
e) conversion sous forme métallique du composant d'or du composé de précurseur d'Au.

14. Procédé selon la revendication 13, **caractérisé en ce que**

a) une première solution d'un composé de précurseur de Pd et/ou d'un composé de précurseur d'Au est mise à disposition ;
b) une deuxième solution d'un composé de précurseur de Pd et/ou d'un composé de précurseur d'Au est mise à disposition, la première solution provoquant une précipitation du/des composant(s) de métal noble du/des composé(s) de précurseur(s) de la deuxième solution et inversement ;
c) la première et la deuxième solution sont appliquées sur le support de catalyseur.

15. Procédé selon la revendication 14, **caractérisé en ce que** le composé de précurseur de la première solution est acide et celui de la deuxième solution est alcalin.

16. Utilisation d'un catalyseur selon l'une quelconque des revendications 9 à 12 en tant que catalyseur dans la synthèse d'alcénylalcanoates, en particulier dans la synthèse de monomère d'acétate de vinyle, en particulier dans l'oxydation en phase gazeuse d'éthylène et d'acide acétique en monomère d'acétate de vinyle.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 565952 A1
- EP 634214 A1
- EP 634209 A1
- EP 634208 A1
- EP 839793 A1
- WO 1998018553 A1
- WO 2000058008 A1
- WO 2005061107 A1
- GB 1229749 A
- DE 4405876 A1
- EP 0723810 A1
- JP H01123631 A
- US 2656323 A
- WO 2005065821 A
- US 5066365 A

**In der Beschreibung aufgeführte Nicht-Patentliteratur**

- **HOLLEMANN WIBERG.** Lehrbuch der anorganischen Chemie. de Gruyter, 2007
- Römpp Lexikon Chemie. Georg Thieme Verlag
- *J. Am. Chem. Soc.,* 1938, vol. 60, 309
- **E.P. BARRET ; L.G. JOINER ; P.P. HAIENDA.** *J. Am. Chem. Soc.,* 1951, vol. 73, 373
- Rahmen der vorliegenden Erfindung geltende Definition des Begriffes Bentonit ist in Römpp. Lexikon Chemie. Georg Thieme Verlag